Europäisches Patentamt

European Patent Office

Office européen des brevets

(11) Numéro de publication: **0 366 549**
**A1**

## (12) DEMANDE DE BREVET EUROPEEN

(21) Numéro de dépôt: 89402948.7

(22) Date de dépôt: 25.10.89

(51) Int. Cl.5: **C12N 15/31 , C12N 9/88 , A61K 39/10**

(30) Priorité: 25.10.88 FR 8813951

(43) Date de publication de la demande:
02.05.90 Bulletin 90/18

(84) Etats contractants désignés:
AT BE CH DE ES FR GB GR IT LI LU NL SE

(71) Demandeur: **INSTITUT PASTEUR**
**25-28, rue du Docteur Roux**
**F-75724 Paris Cédex 15(FR)**

(72) Inventeur: **Danchin, Antoine**
**60, Rue de Reuilly**
**F-75012 Paris(FR)**
Inventeur: **Glaser, Philippe**
**28, Rue Chapon**
**F-75003 Paris(FR)**
Inventeur: **Krin, Evelyne**
**73, Rue Béranger**
**F-92700 Colombes(FR)**
Inventeur: **Barzu, Octavien**
**30, Rue Saussaye**
**F-91300 Massy(FR)**
Inventeur: **Ladant, Daniel**
**24, Rue Ampère**
**F-94230 Cachan(FR)**
Inventeur: **Ullmann, Agnès**
**3 Rue Paul Dupuy**
**F-75016 Paris(FR)**

(74) Mandataire: **Peaucelle, Chantal et al**
**S.C. Ernest Gutmann - Yves Plasseraud 67,**
**boulevard Haussmann**
**F-75008 Paris(FR)**

(54) Dérivés d'adényl cyclase et leurs utilisations biologiques.

(57) Les dérivés d'adényl cyclase sont des dérivés atoxiques dans lesquels un ou plusieurs acides aminés sont différents de ceux présents dans la protéine toxique, les anticorps formés contre ces dérivés reconnaissant, toutefois, la protéine toxique. Ces dérivés sont utilisables comme vaccins.

## DERIVES D'ADENYL CYCLASE ET LEURS UTILISATIONS BIOLOGIQUES

L'invention a pour objet des dérivés d'adényl cyclase, leur préparation par mutagénèse et leurs utilisations biologiques, en particulier comme vaccins.

Les vaccinations contre les infections aïgues causées chez les vertébrés par des bactéries pathogènes du genre Bordetella ou Bacillus anthracis, font appel le plus généralement à l'utilisation de bactéries entières virulentes ou atténuées.

Toutefois, ces vaccins ne sont pas nécessairement dépourvus de toxicité en raison du processus même des maladies provoquées par ces bactéries.

En effet, souvent ce sont des protéines produites par les bactéries, et non les bactéries elles-mêmes, qui sont responsables de la virulence. Même après la mort bactérienne, ces protéines peuvent être responsables de nombreux effets pathologiques.

Dans la pathologie induite par B.pertussis interviennent de façon primordiale une hémagglutinine filamenteuse (FHA) ainsi que deux toxines qui entraînent une augmentation dérégulée de la production d'AMP cyclique pour les cellules hôtes. Il s'agit de la pertussis toxine (Ptx ou LPF) qui active essentiellement la cyclase de l'hôte, et de l'adényl cyclase, activée par la calmoduline (AC).

Enfin, une hémolysine est associée à l'adényl cyclase.

L'adényl cyclase de B.pertussis a été appelée cyclolysine pour indiquer ce fait.

Compte tenu des ces observations, les inventeurs ont recherché des moyens de vaccination dans lesquels n'interviennent plus les bactéries de souches virulentes ou atténuées, mais les protéines produites modifiées de manière appropriée afin de détruire leur toxicité originelle. L'adényl cyclase a plus spécialement retenu l'attention à cet égard.

L'invention a pour but de fournir de nouveaux dérivés de l'adényl cyclase, ces dérivés étant dépourvus d'activité toxique mais étant capables de former des anticorps reconnaissant également les protéines toxiques.

Elle vise également à fournir un procédé d'obtention de ces dérivés par mutagénèse in vitro des gènes codant pour l'adényl cyclase toxique.

Un autre but de l'invention consiste dans la fourniture de vaccins assurant une action protectrice efficace contre les infections produites par des agents pathogènes.

Les dérivés d'adényl cyclase de l'invention sont caractérisés en ce qu'ils comportent un ou plusieurs acides aminés différents de ceux de la protéine toxique en leur conférant un caractère atoxique, ces acides aminés se trouvant impliqués dans le mécanisme catalytique et/ou dans le mode d'activation par la calmoduline et/ou le transfert de l'information du site activateur au site catalytique de l'adényl cyclase et n'affectant pas de manière notable la configuration tridimensionnelle de la protéine toxique, de sorte que les anticorps formés contre les dérivés atoxiques reconnaissent également la protéine toxique.

Une autre caractéristique de l'invention concerne la protéine entière dont les acides aminés responsables de l'activité hémolytique ont été altérés ou délétés.

Le ou les acides aminés de remplacement sont choisis au vu de leurs propriétés physico-chimques, de manière à détruire la toxicité sans affecter notablement la forme de la protéine, tout au moins en ce qui concerne sa reconnaissance par le système immunitaire.

En possédant la même géométrie que la protéine toxique, ces dérivés jouent avantageusement le rôle de leurre pour le système immunitaire sans, toutefois, exercer d'activité catalytique toxique.

Parmi les propriétés permettant d'intervenir sur le caractère toxique originel de la protéine et de le détruire, on citera notamment la taille des acides aminés, leur caractère hydrophobe ou hydrophile, leur charge, leur propension à former ou à détruire les structures secondaires de la séquence d'acides aminés, leur action sur la structure de l'eau, leur caractère rupteur ou formateur d'hélice, leur interaction avec tout ou partie de l'ATP intervenant comme substrat ou avec la calmoduline.

Selon un mode préféré de réalisation de l'invention, les dérivés d'adenyl cyclase renferment un ou avantageusement au moins deux acides aminés différents dans le site catalytique c'est-à-dire dans le domaine responsable de la production d'AMP cyclique (AMPc). Cette disposition permet de supprimer la production d'AMPc ou de la rendre minime tout en conservant les propriétés immunogènes de la protéine.

Le ou les acides aminés différents dans le site catalytique sont choisis plus spécialement de manière à modifier la structure de l'eau ou l'interaction de la protéine avec l'eau ou avec le phosphate, la base nucléique et/ou le sucre de l'ATP, de telle sorte que l'activité ou le contrôle de l'activité du site soit détruit.

Il s'agit par exemple d'acides aminés hydrophobes remplaçant des acides aminés hydrophiles de même taille ou l'inverse, d'acides aminés ayant perdu leur charge électrique ou de charge électrique modifiée, ou encore d'acides aminés de taille différente qui empêcheront la mise en place du substrat ATP, ou la réaction de cyclisation en AMPc.

On citera, à titre d'exemple, le remplacement d'une ou au moins deux lysines qui interagissent avec l'ATP par des acides aminés de même volume, hydrophiles mais dépourvus de charge tel que la glutamine (Q).

Au sein de la séquence peptidique catalytique, le coeur GLNVHAKS peut être modifié, et G e A remplacés chacun par l'un des acides aminés suivants M, L, I, V ou F. K, comme indiqué, peut être remplacé par Q mais aussi par N, D et E, ou encore M, L, I, V.

Les significations des lettres utilisées communément pour désigner les acides aminés ci-dessus et ceux indiqués dans la suite sont donnés en fin de description.

Le domaine responsable de l'activité hémolytique peut, dans la protéine entière, avantageusement être délété par exemple en coupant le gène aux sites Bgl II (AGATCT) et en délétant le fragment correspondant. Cela revient à enlever la portion de la protéine située entre EI xxx et EI xxx.

En variante, ou comme disposition supplémentaire, on peut tirer parti du fait que la protéine ne contient pas de résidu cystéine pour y introduire cet acide aminé par mutagénèse localisée. Celui-ci pourra ensuite subir un traitement chimique approprié (oxydation par exemple ou réaction avec les réactifs des SH de type métalliques, organomercuriels ou iodo acétamide) dont le résultat sera l'inactivation de la protéine.

Les acides aminés différents peuvent être également choisis parmi des analogues d'acides aminés incorporables par la machine de traduction, le cas échéant chimiquement modifiés. On citera à titre d'exemple la fluorophénylalanine, le bromotryptophane, ou la nor-leucine.

Dans un autre mode de réalisation de l'invention, mis en oeuvre le cas échéant avec le mode préféré ci-dessus, le ou les acides aminés de remplacement sont présents dans le site activateur sous l'effet de la calmoduline, et sont choisis en fonction des mêmes paramètres et ce, de manière à permettre une altération de la liaison avec la molécule activatrice de l'hôte.

Ainsi, un résidu acide aspartique hydrophile chargé négativement et rupteur d'hélice remplacera avantageusement un résidu tryptophane de plus grosse taille et hydrophobe et formateur d'hélice, dans la région de liaison à la calmoduline.

Dans encore un autre mode de réalisation, mis en oeuvre seul, ou en combinaison avec au moins l'un de ceux qui précèdent, le ou les acides aminés de remplacement, tels que considérés ci-dessus, sont présents dans la zone responsable du transfert de l'information du site activateur au site catalytique.

De préférence, les dérivés de l'invention sont tels qu'obtnus par expression dans un organisme hôte d'un gène codant pour une protéine à activité adényl cyclase, ce gène ayant été modifié in vitro, après clonage, par mutagénèse dirigée.

Outre les éléments de mutagénèse décrits ci-après, on peut aussi opérer par délétion de régions connues du gène, ou par insertion d'oligonucléotides de synthèse, de manière à détruire un sité doué d'activité.

Les dérivés de l'invention sont plus spécialement des mutants d'adényl cyclase de Bordetella responsables de pathologies respiratoires des vertébrés telles que B.pertussis et B.parapertussis chez l'homme et B.bronchiseptica et B.avium chez l'animal.

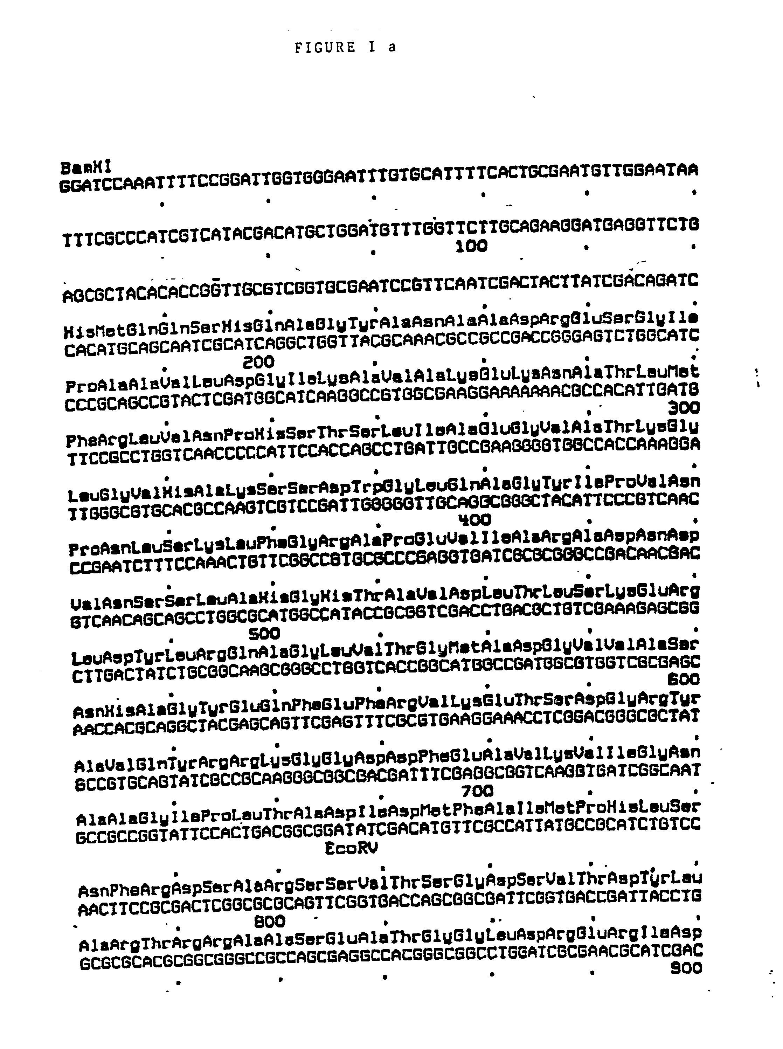

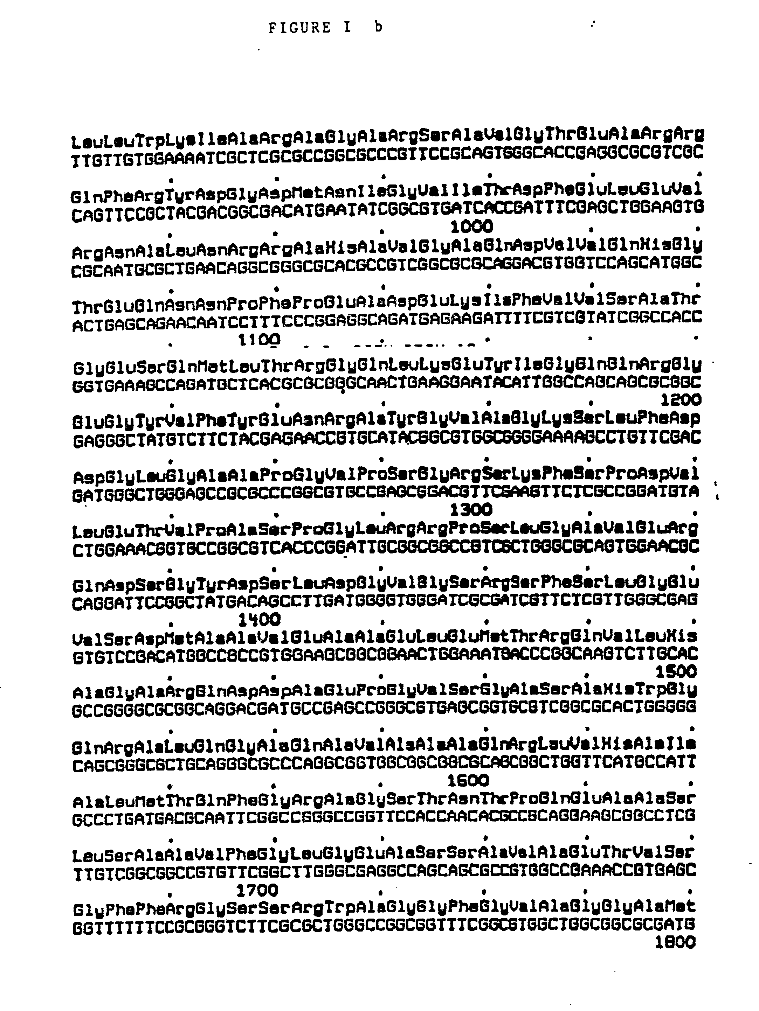

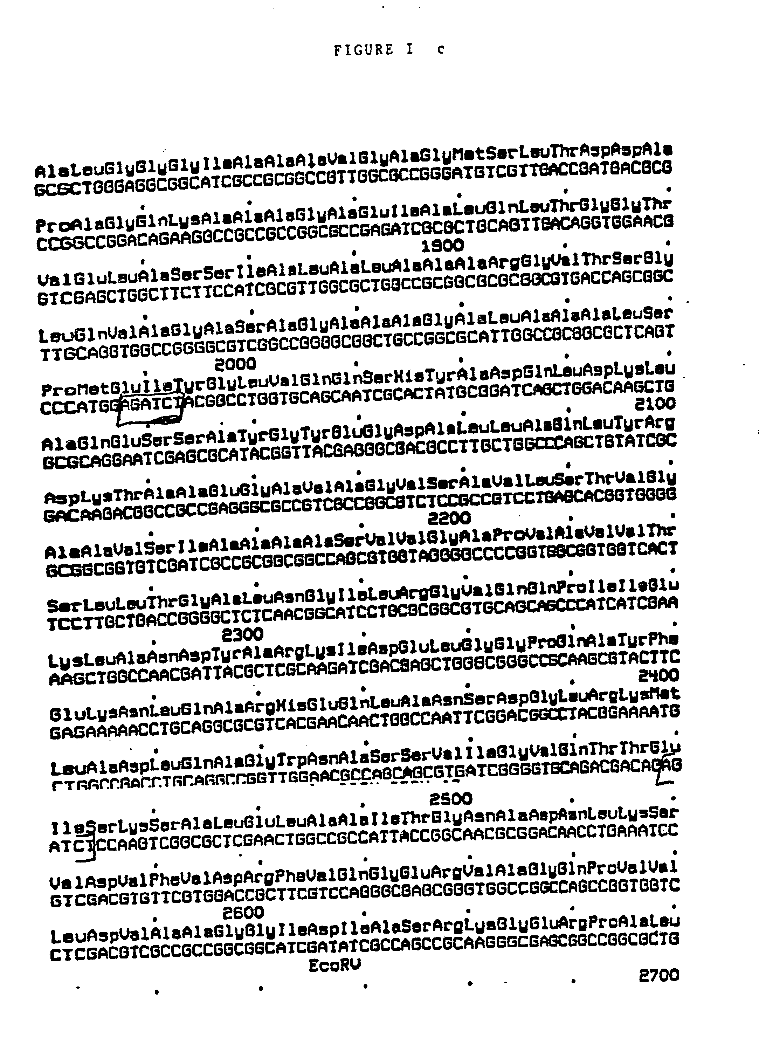

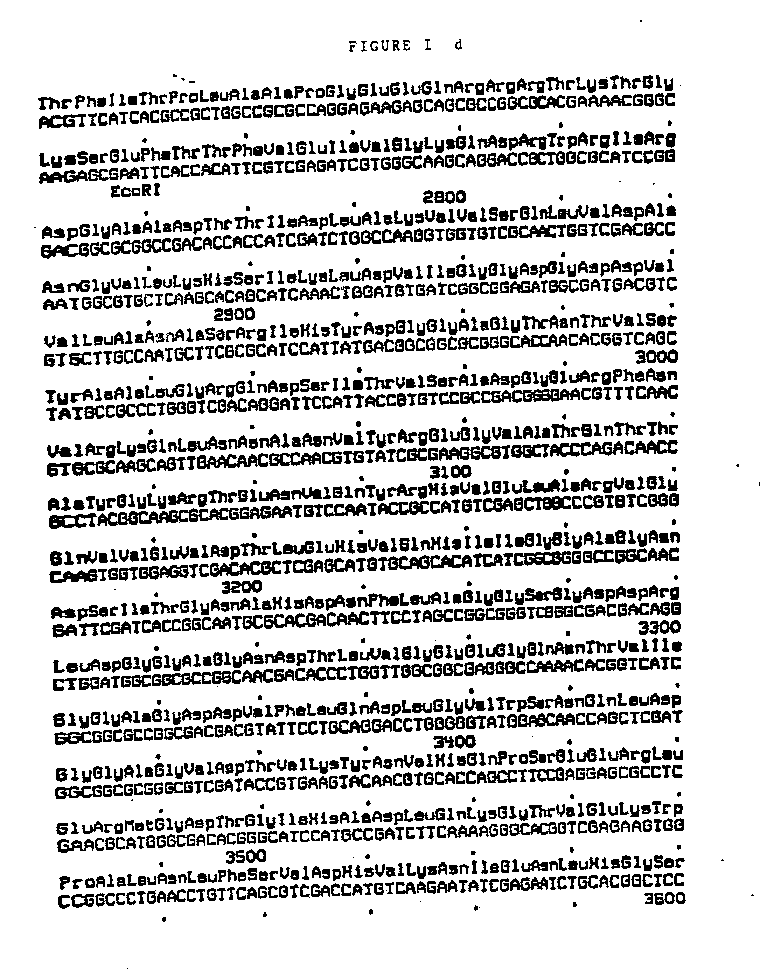

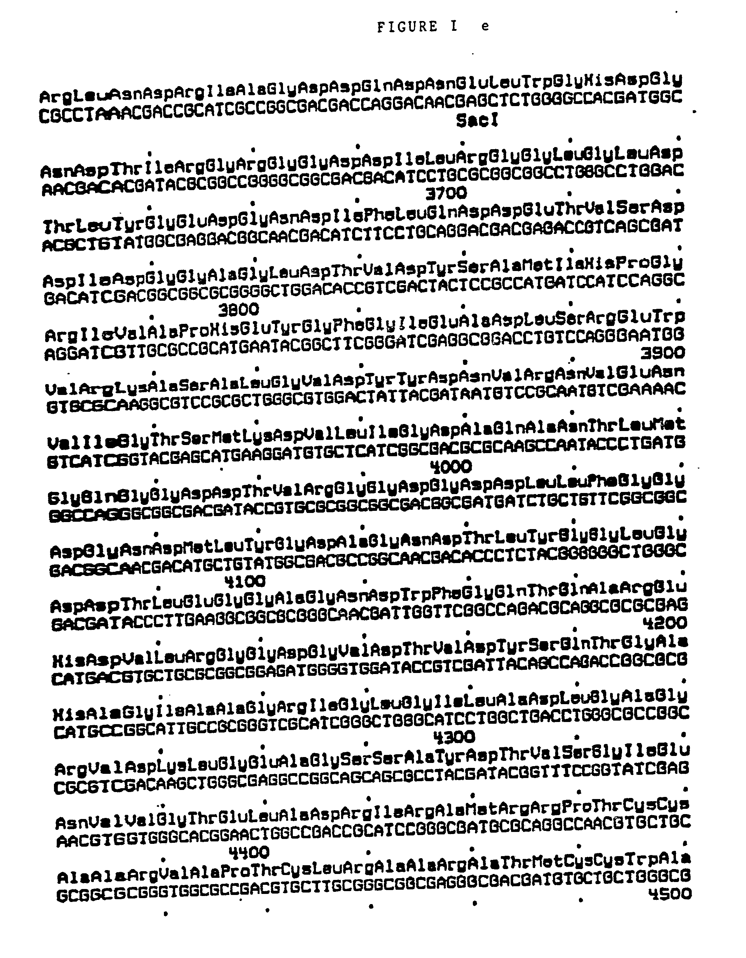

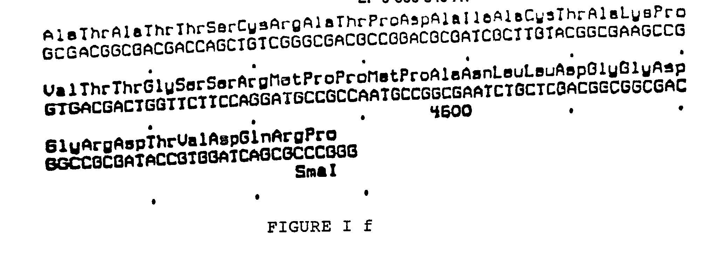

La partie active de l'adényl cyclase toxique de B.pertussis exprimée par le gène cloné présente la séquence (I) donnée sur les figures 1a à 1f. Cette séquence est rapportée dans la demande EP 88/401.935.7 du 25.7.88.

La protéine correspondant à la séquence (I), qui contient 1706 acides aminés, comprend deux domaines présentant deux activités principales.

Les 400 à 500 acides aminés de la partie N-terminale sont responsables de l'activité adényl cyclase et les 1300 à 1200 de la partie carboxy-terminale de l'activité hémolytique et des propriétés de secrétion de la protéine.

Dan la partie catalytiquement active pour la synthèse d'AMPc, l'adényl cyclase apparaît organisée en deux domaines :
le domaine N-terminal de 25kDa qui porte le site catalytique et le domaine C-terminal de 12kDa qui porte le site principal de liaison à la calmoduline, plus spécialement la séquence correspondant aux résidus 235-254.

Dans une autre forme de réalisation de l'invention, les dérivés d'adényl cyclase sont des mutants d'une adényl cyclase du type de celle exprimée par B.anthracis. La séquence de nucléotides du gène codant pour cette adényl cyclase et la séquence de cette dernière sont rapportées dans la demande France déposée ce jour aux noms des demandeurs, intitulée "SEQUENCES DE NUCLEOTIDES EXPRIMANT L'ADENYL CYCLASE DE B. ANTHRACIS, PROTEINES AYANT L'ACTIVITE DE CETTE ADENYL CYCLASE ET APPLICATIONS BIOLOGIQUES".

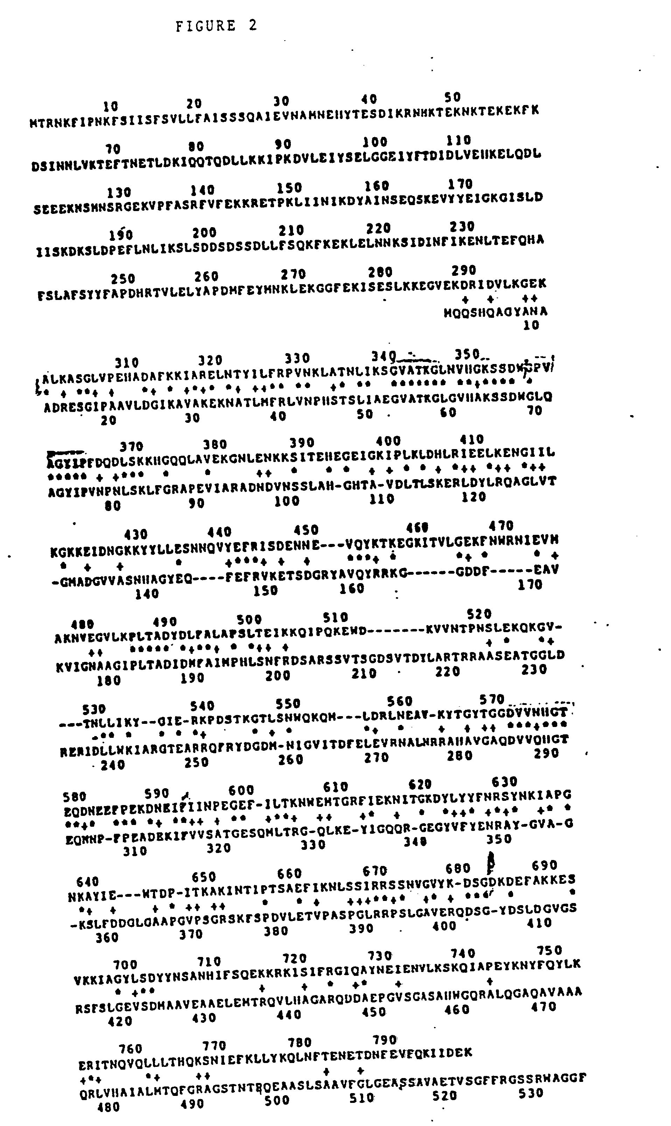

La séquence d'acides aminés de l'adényl cyclase de B. anthracis présente plusieurs régions communes avec celle secrétée par B. pertussis - (voir figure 2).

La similitude la plus importante correspond au peptide de 24 acides aminés (de la position 342 à la position 365 chez l'adenyl cyclase de B. anthracis : GVATKGLNVHGKSSDWGPVAGYIP). Cette séquence contient cinq résidus gly et la séquence noyau G --- GKS (AKS chez B.pertussis) que l'on retrouve souvent dans les protéines ayant une affinité pour les nucléotides.

Deux autres régions présentent une similitude plus faible. Elles correspondent chez B.anthracis aux domaines s'étendant des positions 487 à 501 (PLTADYDLFALAPSL) d'une part et 573 à 594 (DVVNHGTEQDNEEFPEKDNEIF)d'autre part.

En variante, les dérivés de l'invention sont tels qu'obtenus part traitement chimique de la séquence d'adényl cyclase modifiée.

L'invention vise également les anticorps polyclonaux dirigés contre ces adényl cyclases modifiées, ainsi que les anticorps monoclonaux capables de les reconnaître spécifiquement.

Conformément à l'invention, les dérivés d'adényl cyclase définis plus haut sont obtenus par mutagénèse in vitro du gène cloné exprimant la protéine toxique, suivie d'expression du gène dans des hôtes appropriés, par exemple E.coli, B.avium ou Alcaligenes eutrophus avec éventuellement un traitement chimique subséquent (cas particulier des protéines à cystéine par exemple). Cette expression s'effectue avec des rendements élevés en particulier chez E. coli. Le gène peut être réintroduit dans la bactérie pathogène afin d'y exprimer la protéine atoxique. La bactérie ainsi modifiée fait également partie de l'invention.

Le procédé d'obtention de dérivés d'adényl cyclase atoxiques selon l'invention comprend la mutagénèse in vitro du gène cloné exprimant l'adényl cyclase toxique, cette mutagénèse étant effectuée dans la région codant pour le site catalytique et/ou pour le site activateur par la calmoduline et/ou pour le site de transfert de l'information du site activateur au site catalytique et/ou la région responsable de l'activité hémolytique, de manière à introduire un ou plusieurs codons codant pour des acides aminés différents de ceux présents dans la protéine toxique, ou à faire disparaître (déléter) des codons présents dans le gène original capables de lui conférer un caractère atoxique tout en n'affectant pas de manière notable la configuration tridimensionnelle présentée par la protéine toxique, de sorte que les anticorps formés contre les dérivés atoxiques reconnaissant également la protéine toxique.

La mutagénèse porte avantageusement sur les codons codant pour les acides aminés définis ci-dessus.

Selon un mode préféré de réalisation de l'invention, on met en oeuvre le système de mutagénèse in vitro d'Amersham ou de toute autre compagnie proposant des systèmes du même type comportant l'utilisation d'un oligonucléotide mutant pour diriger la mutagénèse. Cet oligonucléotide est construit de manière à comporter le codon capable de coder pour l'acide aminé que l'on souhaite introduire. Le codon final souhaité peut être introduit en une seule étape, ou en ayant recours à l'introduction intermédiaire d'un autre codon puis dans une étape ultérieure, à sa mise en place.

En variante, il est aussi possible d'utiliser un oligonucléotide mutant comportant une ambiguité et capable de coder pour deux acides aminés différents. De même, on peut utiliser un oligonucléotide où il manque un ou plusieurs codons, de façon à produire une délétion.

L'oligonucléotide mutant renferme avantageusement au moins 15 nucléotides, notamment de 20 à 25. Conformément à la technique classique de mutagénèse in vitro, utilisant le kit Amersham, on a recours aux étapes suivantes :
- hybridation de l'oligonucléotide mutant avec une séquence d'ADN d'une matrice d'ADN monobrin construite de manière appropriée à partie d'un vecteur par mutagénèse in vitro : on utilise avantageusement, comme vecteur à ADN monobrin, un phage, notamment dérivé de M13,
- synthèse en présence d'α thio dCTP et ligation du brin d'ADN mutant avec une polymérase telle que le fragment de Klenow de l'ADN polymérase I de E.Coli.
- élimination de l'ADN monobrin (non mutant), par exemple par filtration, - coupure du brin d'ADN non mutant à l'aide d'une enzyme de restriction, telle que NciI,
- digestion du brin non mutant à l'aide par exemple d'une exonucléase, notamment l'exonucléase III,
- repolymérisation et ligation de l'ADN partiellement lacunaire,
- transformation de cellules hôtes compétentes avec l'ADN.

Ce procédé présente l'avantage d'obtenir, avec des rendements éléves, le dérivé atoxique. Il est clair toutefois que d'autres modes de réalisation de la mutagénèse par exemple utilisant des sites de restriction pour créer des insertions, des délétions ou des remplacements, peuvent être mis en oeuvre ou que des modifications peuvent être apportées à la méthode basée sur l'utilisation du kit commercialisé par Amersham pour introduire les codons appropriés et obtenir les dérivés d'adényl cyclase atoxiques de l'invention.

Le gène codant pour l'adényl cyclase exprimée par l'un des microorganismes indiqué ci-dessus est avantageusement cloné selon le procédé décrit dans la demande FR 87/10614 du 24 Juillet 1987 aux noms des demandeurs.

Dans ce procédé, on utilise l'interaction adényl cyclase-calmoduline qui se traduit par la production de cAMP. Le clonage du gène est effectué dans une souche réceptrice déficiente en adényl cyclase, portant un plasmide exprimant de hauts niveaux de calmoduline. On observera que les gènes qui coopèrent dans ce procédé de clonage, à savoir celui qui code pour l'adényl cyclase et celui qui code pour la calmoduline sont d'origines différentes.

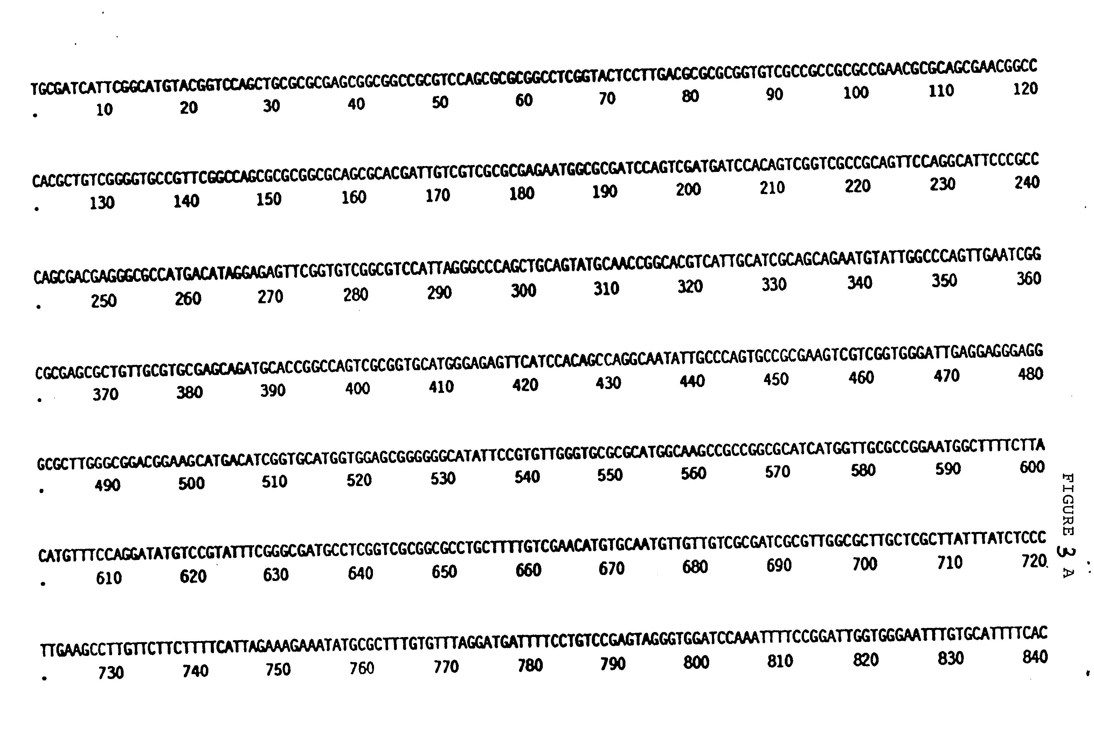

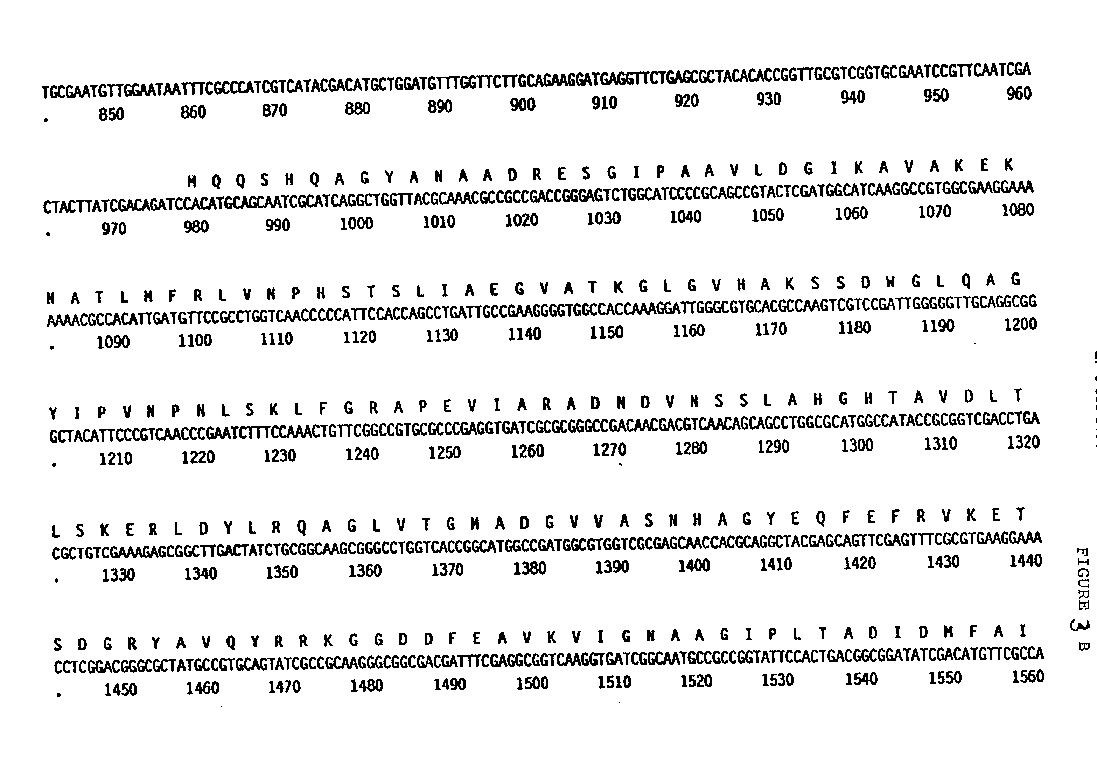

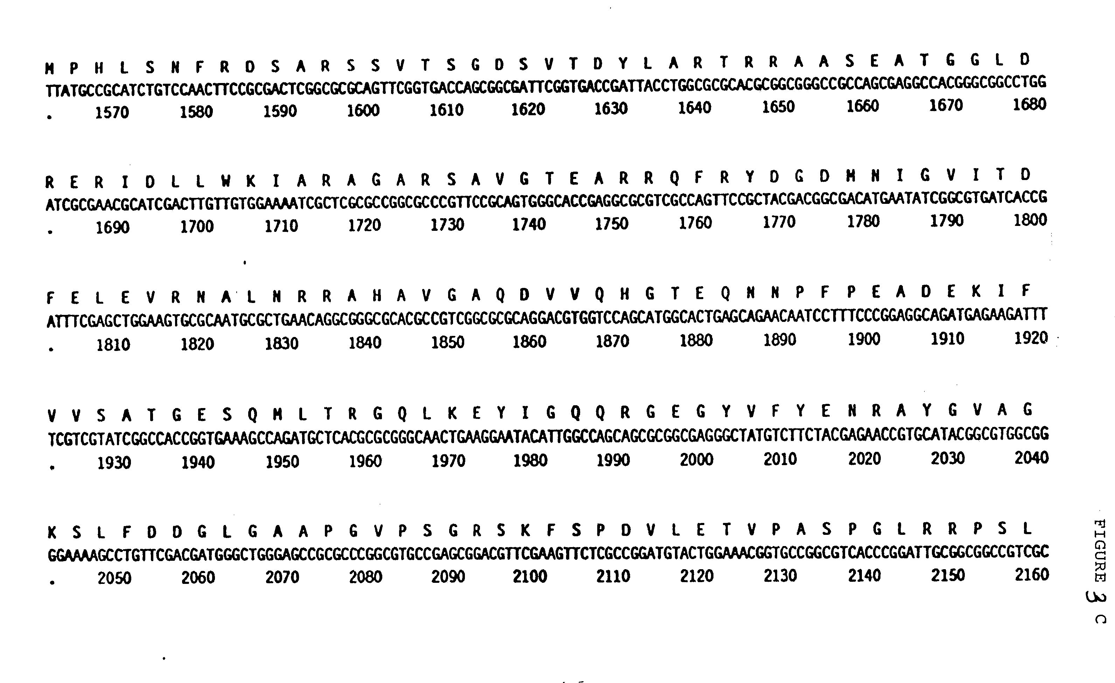

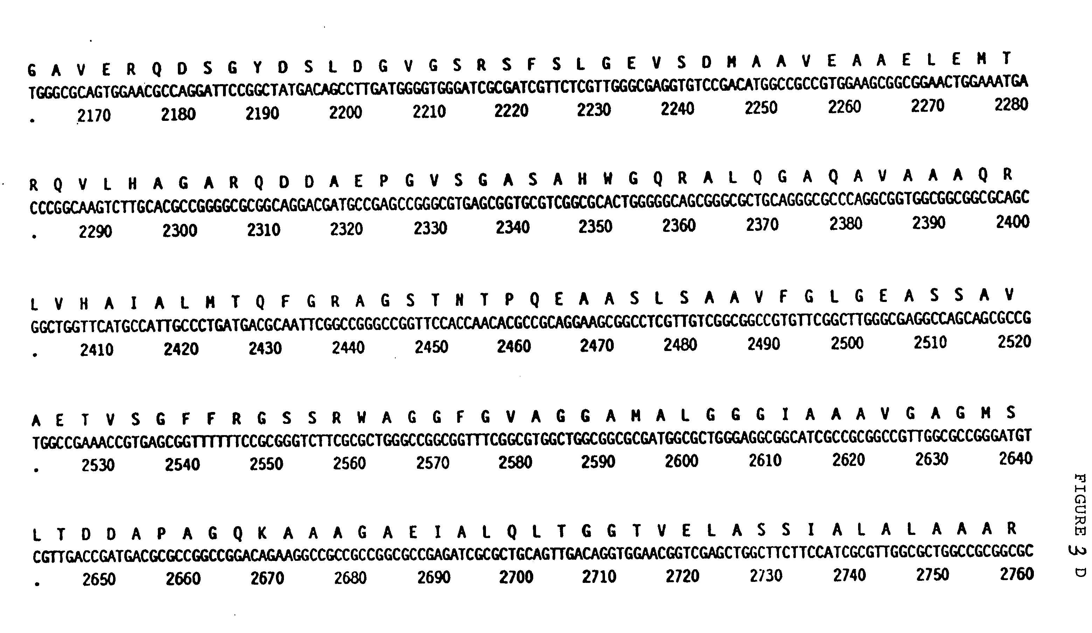

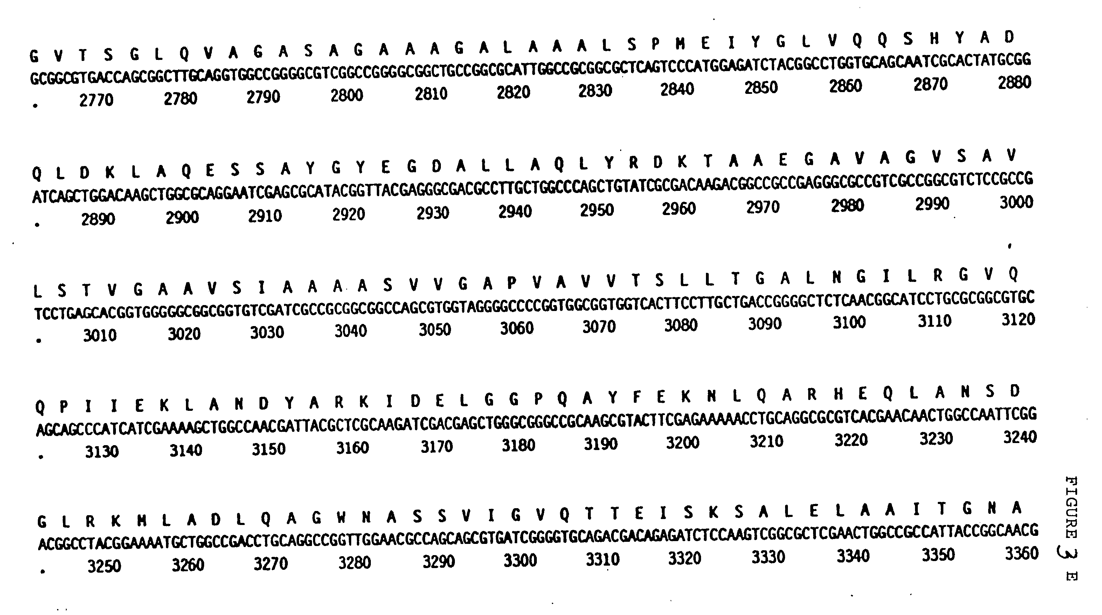

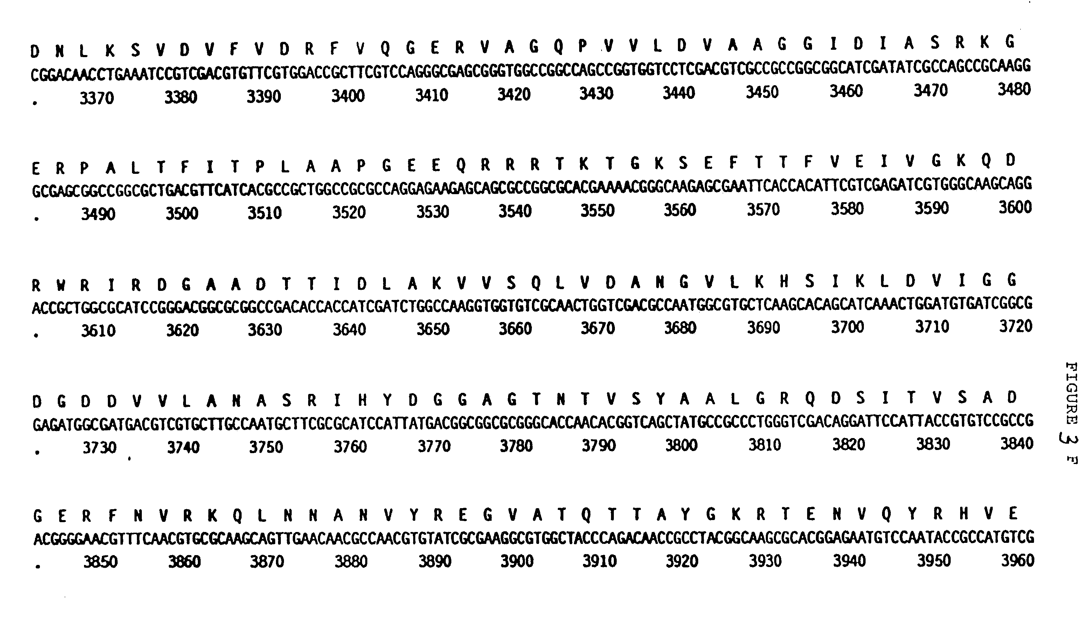

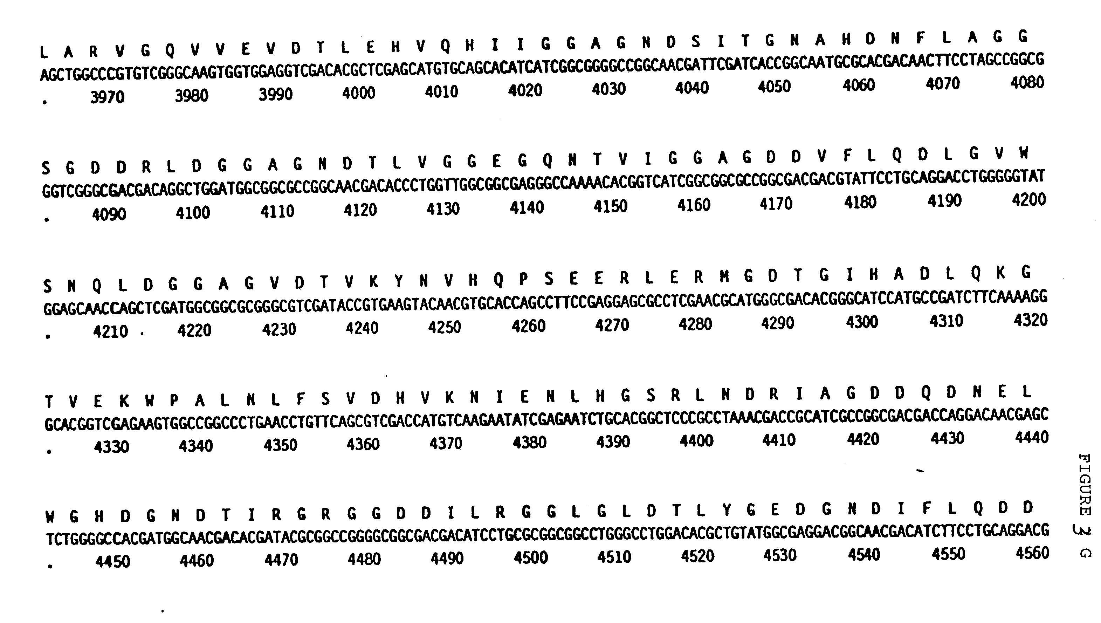

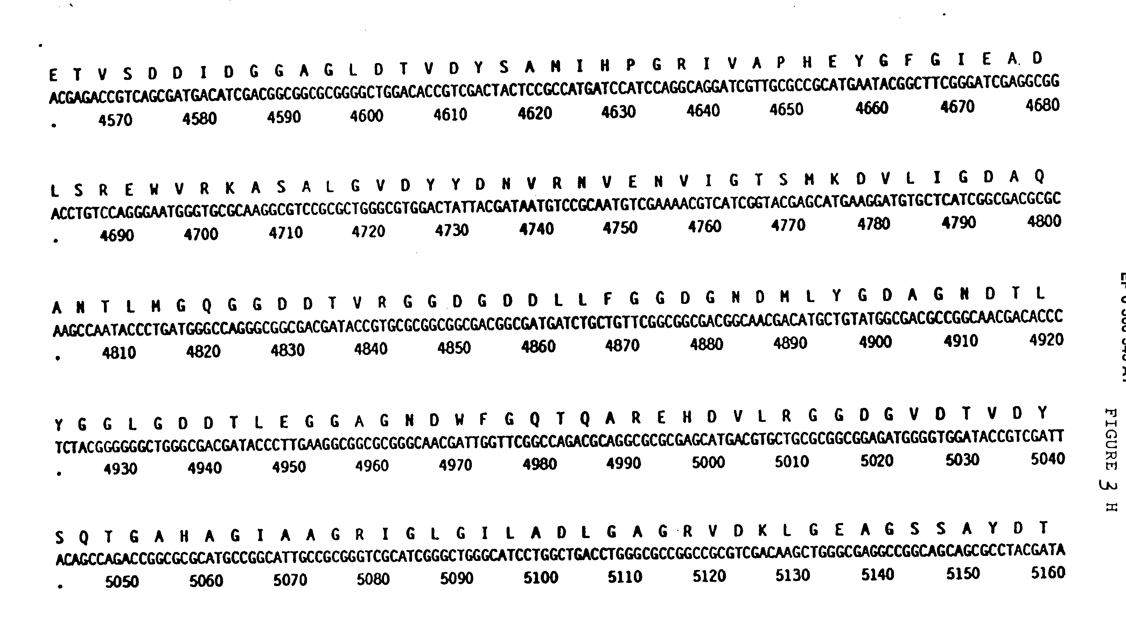

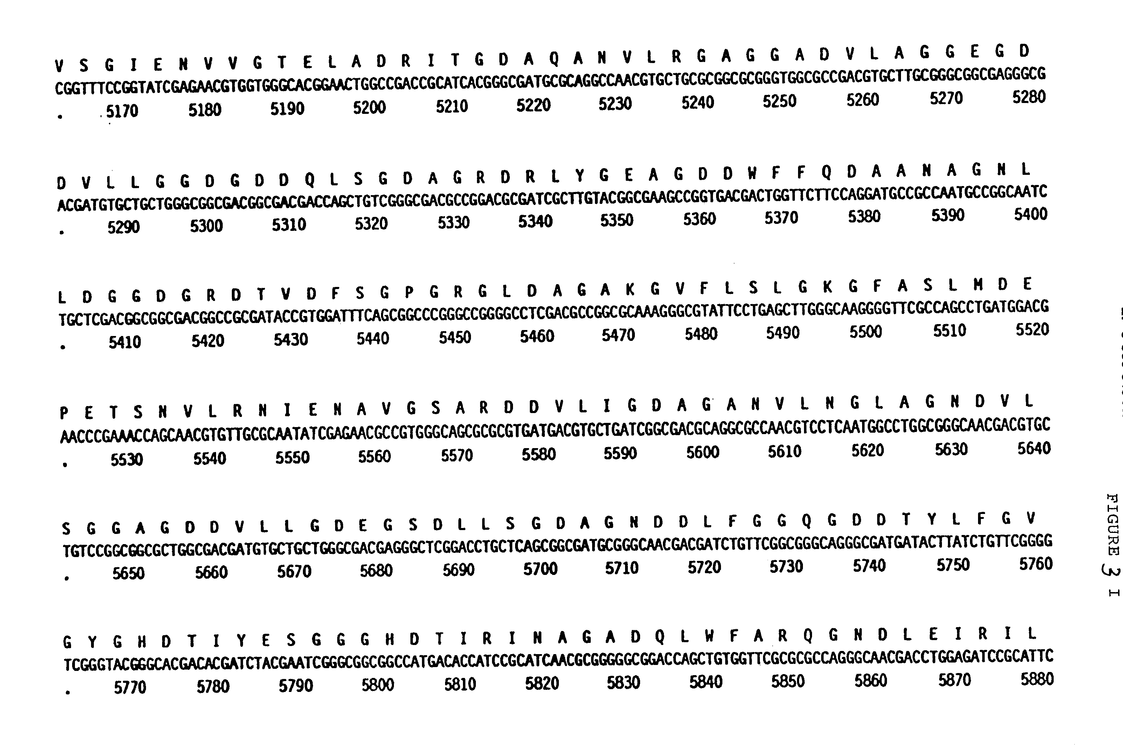

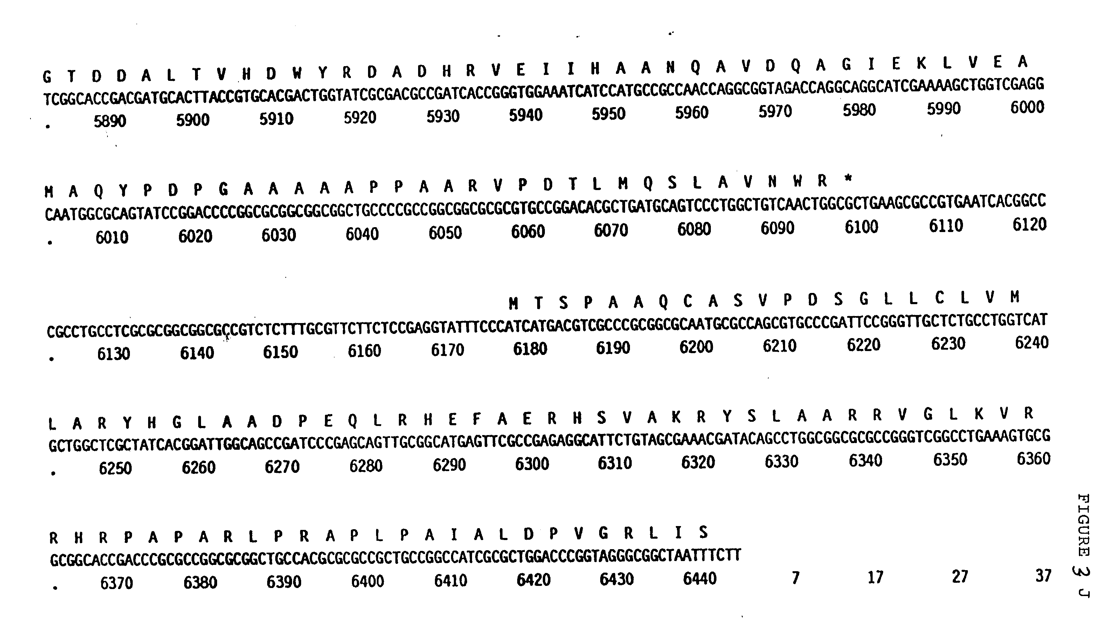

Le fragment d'ADN cloné de la partie active du gène codant pour l'adényl cyclase de B.pertussis, utilisable dans le procédé de mutagénèse de l'invention, répond à la séquence représentée également sur les figures 1a à 1f ou encore sur les figures 3a à 3j.

La partie catalytique pour la synthèse d'AMPc du fragment d'ADN comprend la séquence de nucléotides localisée entre les positions 0 et 2050, la position 0 correspondant au site de restriction BamHI.

Toute séquence de nucléotides qui s'hybride avec la séquence de nucléotides des figures 1a à 1f ou 3a à 3j et code pour une protéine à activité adényl cyclase, que cette séquence soit obtenue par transcription enzymatique inverse de l'ARN correspondant ou par synthèse chimique est utilisable dans le procédé selon l'invention aux fins de mutagénèse.

L'invention vise également les séquences de nucléotides comportant ou constituées par le gène codant pour au moins la partie active d'une adényl cyclase, mais mutées de manière à coder pour un dérivé d'adényl cyclase tel que défini ci-dessus ainsi que les oligonuléotides mutants.

Ainsi, par rapport au fragment d'ADN des figures 1a à 1f une séquence nucléotidique selon l'invention comporte un codon CAA à la place du codon AAA en position 58, ou encore un codon CAG à la place d'un codon AAG en position 65. De préférence, ces oligonucléotides ne pourront pas permettre une hybridation non spécifique avec le vecteur M13.

Les oligonucléotides mutants comprennent par exemple un codon CAA à la place d'un codon AAA en position 58 et comprennent tout ou partie de la séquence GTG GCC ACC CAA GGA TTG G.

Il peut s'agir également d'oligonucléotides avec un codon CAG à la place d'un codon AAG en position 65 et comprenant tout ou partie de la séquence GTGCACGCCCAGTCGTCCG.

D'autres oligonucléotides comprennent à la place d'un codon TGG en position 242, un codon GAC et répondent à tout ou partie de la séquence CTTGTTGGACAAAATCGC, ou un codon GAC à la place du codon GAC de l'oligonucléotide précédent.

Un mélange d'oligonucléotides peut permettre d'obtenir plusieurs mutants dans une seule expérience.

En variante, on obtient les dérivés atoxiques de l'invention en soumettant la séquence protéique d'une adényl cyclase ou tout au moins sa partie active à un traitement chimique par exemple les traitements d'inactivation utilisés en vaccination, par exemple pour les vaccinations tétaniques.

Les essais de toxicologie réalisés sur les dérivés de protéines de l'invention ont démontré leur absence de toxicité.

Les propriétés fortement immunogènes des dérivés de l'invention sont donc avantageusement mises à profit pour l'élaboration de vaccins.

Les vaccins de l'invention sont caractérisés en ce qu'il s'agit de vaccins moléculaires renfermant au moins un dérivé atoxique d'adényl cyclase tel que défini ci-dessus, en association avec un véhicule pharmaceutique.

Ils peuvent également renfermer la bactérie porteuse des gènes mutés et rendue ainsi atoxique.

La vaccination est effectuée selon les doses et avec les formes d'administration habituelles.

D'une manière avantageuse, les dérivés d'adényl cyclase de l'invention donnent lieu à une réaction immunologique croisée avec des anticorps dirigés contre l'adényl cyclase de B.pertussis. Des compositions de vaccins avantageuses comprennent le dérivé d'adényl cyclase de l'invention associé à une préparation de bactéries tuées de B.pertussis utilisée comme adjuvant..

Pour illustrer l'invention, on rapporte ci-après des exemples de préparation de mutants d'adényl cyclase de B.pertussis. Dans ces exemples, on se réfère aux figures 1a à 1f et 3a à 3j, qui représentent, les séquences de nucléotides de la partie active du gène codant pour l'adényl cyclase respectivement de B.pertussis et de B.anthracis, clonée dans E.coli et la séquence d'acides aminés correspondante exprimée, et à la figure 2 la séquence complète du polypeptide de l'adényl cyclase de B.anthracis (ligne supérieure) et la séquence polypeptidique N-terminale de l'adényl cyclase de B.pertussis (ligne inférieure).

EXEMPLES :

1/ - Clonage du gène codant pour l'adényl cyclase de B.pertussis.

Des souches cya⁻, restriction⁻ de E.coli sont transformées par un plasmide porteur d'un gène spécifiant la synthèse de calmoduline tel que le plasmide pVUC-1, ou de tout autre plasmide exprimant la calmoduline du groupe d'incompatibilité ColE1 et porteur du gène de résistance à l'ampicilline.

On introduit ensuite dans les souches cya⁻ des vecteurs renfermant de l'ADN de B.pertussis total fragmenté de diverses manières (coupures partielles par des enzymes de restriction EcoRI et SauIIIa, sonication suivie d'addition de "linkers" EcoRI par exemple).

En raison des problèmes posés par l'incompatibilité d'autres plasmides ayant le réplicon ColEI le clonage de fragments d'ADN total de B.pertussis partiellement coupé par l'enzyme Sau|||a a été effectué dans le vecteur compatible pACYC184, au site unique BamH1. Les souches réceptrices ont été transformées et étalées sur des boîtes Mc Conkey Maltose. Plusieurs clones fermentant le maltose ont été isolés. Il a ensuite été démontré, que ces clones produisent de l'AMP cyclique. Par ailleurs, les plasmides extraits des souches productrices ont été intégrés par transformation dans des souches qui soit ne contiennent pas le plasmide pVUC-1, soit le contiennent. Dans le premier cas, les clones sont blancs sur les milieux Mc Conkey maltose, rouges dans le second, confirmant que c'est bien la présence des plasmides permettant la synthèse de calmoduline qui a permis le clonage.

2/ - Mutagénèse in vitro du gène cloné codant pour l'adényl cyclase.

En opérant comme décrit par Carter et al dans Nucl. Acids. Res. 13, 4431-43, 1985, on remplace un codon AAA (codant pour K) par un codon CAA (codant pour Q) en position 58 en utilisant l'oligonucléotide GTGGCCACCCAAGGATTGG. De même, on remplace un codon AAG (codant pour K) en position 65 par un codon CAG (codant pour Q) en utilisant l'oligonucléotide GTGCACGCCCAGTCGTCCG.

Les deux modifications sont effectuées sur le fragment d'ADN BamHI-EcoRV cloné dans le phage M13tg130.

Le codon TGG en position 242 (codant pour un tryptophane) est tout d'abord modifié en un codon GAC (codant pour l'acide aspartique) en utilisant l'oligonucléotide CTTGTTGGACAAAATCGC. Le codon GAC est ensuite modifié en un codon GAG (codant pour l'acide glutamique) en utilisant l'oligonucléotide CTTGTTGGAGAAAATCGC. On observera que, dans ce cas, la mutagénèse porte sur un codon qui ne se trouve pas dans une zone de similitude avec les séquences de nucléotides d'autres gènes codant pour un adényl cyclase, plus spécialement celle de B. anthracis.

Les modifications sur la position 242 ont été effectuées sur le fragment d'ADN EcoRV-EcoRI cloné dans le phage M13tg130.

Pour chaque mutagénèse, on contrôle sur le fragment muté l'absence de tout autre mutation par la méthode de séquençage avec un didéoxynucléotides.

3/ - Obtention des dérivés atoxiques d'adényl cyclase.

Par expression du gène muté dans une souche de E.coli selon les techniques habituelles, on obtient l'adényl cyclase atoxique qui est ensuite purifiée.

On constate que le remplacement du tryptophane par l'aspartate réduit d'un facteur de 1000 l'activabilité de l'enzyme par la calmoduline, son activité de base étant maintenue.

Le remplacement de la lysine en position 65 diminue l'activité de base intrinsèque de l'enzyme d'un facteur d'au moins 100.

Les lettres pour désigner les acides aminés présentent les significations suivantes :
D Acide aspartique
E Acide glutamique
A Alanine
R Arginine
N Asparagine
C Cystéine
Q Glutamine
G Glycine
H Histidine
I Isoleucine
L Leucine
K Lysine
M Méthionine
F Phenylalanine
P Proline
S Sérine
T Thréonine
W Tryptophane
Y Tyrosine
V Valine

Revendications

1. Dérivés d'adényl cyclase, caractérisés en ce qu'ils comportent un ou plusieurs acides aminés différents de ceux de la protéine toxique, en lui conférant un caractère atoxique, ces acides aminés se trouvant dans le site catalytique et/ou dans le site activateur par la calmoduline et/ou dans le site de transfert de l'information du site activateur au site catalytique et/ou dans la région hémolytique, et n'affectant pas de manière notable la configuration tridimensionnelle de la protéine toxique, de sorte que les anticorps formés contre les dérivés atoxiques reconnaissent également la protéine toxique.

2. Dérivés selon la revendication 1, caractérisés en ce que le ou les acides aminés de remplacement présentent des tailles et/ou un caractère hydrophobe ou hydrophile et/ou une charge et/ou une propension à former ou à détruire les structures secondaires de la séquence d'acides aminés et/ou une action sur la structure de l'eau et/ou une interaction avec tout ou partie de l'ATP intervenant comme substrat et/ou avec la calmoduline différents de celles des acides aminés de la protéine toxique.

3. Dérivés selon la revendication 1 ou 2, caractérisés en ce qu'ils renferment un ou avantageusement au moins deux acides aminés différents dans le site catalytique de l'adényl cyclase.

4. Dérivés selon l'une quelconque des revendications 1 à 3, caractérisés en ce qu'ils comportent une ou au moins deux glutamines ou asparagines, aspartates, ou glutamates à la place de résidus de lysines.

5. Dérivés selon la revendication 3, caractérisés en ce que le noyau de la séquence peptidique catalytique GLNVHAKS est modifié, G et A étant remplacé chacun par l'un des acides aminés suivants : M, L, I, V ou F, K pouvant être remplacé par Q, mais aussi par N et D, E, ou encore M, L, I, V, les lettres utilisées pour désigner les acides aminés ayant les significations suivantes : A: alanine, F: phénylalanine, G: glycine, H: histidine, I: isoleucine, K: lysine, L: leucine, M: méthionine, N: asparagine, S: sérine, et V: valine.

6. Dérivés selon l'une quelconque des revendication 1 à 3, caractérisés en ce qu'ils comportent un ou plusieurs résidus cystéine, le cas échéant chimiquement modifiés.

7. Dérivés selon l'une quelconque des revendications 1 à 3, caractérisés en ce qu'ils renferment un ou plusieurs analogues d'acides aminés incorporables par la machine de traduction, le cas échéant chimiquement modifiés.

8. Dérivés selon l'une quelconque des revendications précédentes, caractérisés en ce que le ou les acides aminés de remplacement sont présents dans le site activateur.

9. Dérivés selon la revendication 1 ou 2, caractérisés en ce qu'ils comportent un résidu aspartate, glutamate, asparagine ou glutamine, à la place d'un résidu tryptophane de la région de liaison à la calmoduline.

10. Dérivés selon l'une quelconque des revendications 1 à 9, caractérisés en ce qu'il s'agit de mutants d'adényl cyclase de Bordetella telles que B.pertussis, B.bronchiseptica, B.avium, et de Bacillus anthracis.

11. Dérivé selon la revendication 10, caractérisé en ce qu'il s'agit d'un mutant de la partie active de l'adényl cyclase de B.pertussis représentée par la séquence (I) des figures 1a à 1f ou (II) des figures 3a à 3j.

12. Dérivés d'adényl cyclase selon la revendication 1 tels qu'obtenus par expression dans un organisme hôte d'un gène codant pour une protéine à activité adényl cyclase, ce gène ayant été modifié in vitro, après clonage, par mutagénèse dirigée.

13. Procédé d'obtention de dérivés d'adényl cyclase atoxiques selon la revendication 1, caractérisés en ce qu'il comprend la mutagénèse in vitro du gène cloné exprimant l'adényl cyclase toxique, cette mutagénèse étant effectuée dans la région codant pour le site catalytique et/ou pour le site activateur par la calmoduline et/ou pour le site de transfert de l'information du site activateur au site catalytique et/ou la région responsable de l'hémolyse, de manière à introduire un ou plusieurs codons codant pour des acides aminés différents de ceux présents dans la protéine toxique, ou de déléter certains codons capables de lui conférer un caractère atoxique, tout en n'affectant pas de manière notable la configuration tridimensionnelle présentée par la protéine toxique, de sorte que les anticorps formés contre les dérivés atoxiques reconnaissent également la protéine atoxique.

14. Procédé selon la revendication 13, caractérisé en ce que les codons mutés codent pour des acides aminés différents de ceux de la protéine toxique définie dans l'une quelconque des revendications 2 à 9.

15. Procédé selon la revendication 14, caractérisé en ce qu'on utilise un oligonucléotide mutant pour diriger la mutagénèse.

16. Procédé selon la revendication 15, caractérisé en ce que l'oligonucléotide mutant comprend un codon CAA à la place d'un codon AAA, en position 58 de l'adényl cyclase de B. pertussis, et comprend tout ou partie de la séquence GTGGCCACCCAAGGATTGG, ou encore qu'il comporte un codon CAG à la place d'un codon AAG en position 65 et comprend tout ou partie de la séquence GTGCACGCCCAGTCGTCCG, ou un codon GAC à la place d'un codon TGG en position 242 et comprend tout ou partie de la séquence CTTGTTGGGACAAAATCGC, ou un codon GAG à la place du codon GAC de l'oligonucléotide précédent.

17. Vaccins induisant la synthèse d'anticorps dotés d'une action protectrice contre les infections à Bordetella et Bacillus anthracis, caractérisés en ce qu'il s'agit de vaccins moléculaires renfermant un dérivé d'adényl cyclase selon l'une quelconque des revendications 1 à 12, en association avec un véhicule pharmaceutique, ou le cas échéant des bactéries entières porteuses des gènes mutés.

18. Compositions de vaccins selon la revendication 17, caractérisées en ce que le dérivé d'adényl cyclase est associé à une préparation de bactéries tuées de B.pertussis utilisées comme adjuvant.

19. Séquence de nucléotides comportant ou constituée par le gène codant pour au moins la partie active d'une adényl cyclase, caractérisée en ce qu'il s'agit d'une séquence d'adényl cyclase mutée de manière à coder pour un dérivé selon l'une quelconque des revendications 1 à 12 et oligonucléotides mutants formés d'une partie de ces séquences.

## FIGURE I a

```
BamHI
GGATCCAAATTTTCCGGATTGGTGGGGAATTTGTGCATTTTCACTGCGAATGTTGGAATAA


TTTCGCCCATCGTCATACGACATGCTGGATGTTTGGTTCTTGCAGAAGGATGAGGTTCTG
                                                  100


AGCGCTACACACCGGTTGCGTCGGTGCGAATCCGTTCAATCGACTACTTATCGACAGATC
HisMetGlnGlnSerHisGlnAlaGlyTyrAlaAsnAlaAlaAspArgGluSerGlyIle
CACATGCAGCAATCGCATCAGGCTGGTTACGCAAACGCCGCCGACCGGGAGTCTGGCATC
          200
ProAlaAlaValLeuAspGlyIleLysAlaValAlaLysGluLysAsnAlaThrLeuMet
CCCGCAGCCGTACTCGATGGCATCAAGGCCGTGGCGAAGGAAAAAAACGCCACATTGATG
                                                          300
PheArgLeuValAsnProHisSerThrSerLeuIleAlaGluGlyValAlaThrLysGlu
TTCCGCCTGGTCAACCCCCATTCCACCAGCCTGATTGCCGAAGGGGTGGCCACCAAAGGA


LeuGlyValHisAlaLysSerSerAspTrpGlyLeuGlnAlaGluTyrIleProValAsn
TTGGGCGTGCACGCCAAGTCGTCCGATTGGGGGTTGCAGGCGGGCTACATTCCCGTCAAC
                                              400
ProAsnLeuSerLysLeuPheGlyArgAlaProGluValIleAlaArgAlaAspAsnAsp
CCGAATCTTTCCAAACTGTTCGGCCGTGCGCCCGAGGTGATCGCGCGGGCCGACAACGAC


ValAsnSerSerLeuAlaHisGlyHisThrAlaValAspLeuThrLeuSerLysGluArg
GTCAACAGCAGCCTGGCGCATGGCCATACCGCGGTCGACCTGACGCTGTCGAAAGAGCGG
                                      500
LeuAspTyrLeuArgGlnAlaGlyLeuValThrGlyMetAlaAspGlyValValAlaSer
CTTGACTATCTGCGGCAAGCGGGCCTGGTCACCGGCATGGCCGATGGCGTGGTCGCGAGC
                                                        600
AsnHisAlaGlyTyrGluGlnPheGluPheArgValLysGluThrSerAspGlyArgTyr
AACCACGCAGGCTACGAGCAGTTCGAGTTTCGCGTGAAGGAAACCTCGGACGGGCGCTAT


AlaValGlnTyrArgArgLysGluGlyAspAspPheGluAlaValLysValIleGlyAsn
GCCGTGCAGTATCGCCGCAAGGGCGGCGACGATTTCGAGGCGGTCAAGGTGATCGGCAAT
                                              700
AlaAlaGlyIleProLeuThrAlaAspIleAspMetPheAlaIleMetProHisLeuSer
GCCGCCGGTATTCCACTGACGGCGGATATCGACATGTTCGCCATTATGCCGCATCTGTCC
EcoRV


AsnPheArgAspSerAlaArgSerSerValThrSerGlyAspSerValThrAspTyrLeu
AACTTCCGCGACTCGGCGCGGCAGTTCGGTGACCAGCGGCGATTCGGTGACCGATTACCTG
                                    800
AlaArgThrArgArgAlaAlaSerGluAlaThrGlyGlyLeuAspArgGluArgIleAsp
GCGCGCACGCGGCGCGGGCCGCCAGCGAGGCCACGGGCGGCCTGGATCGCGAACGCATCGAC
                                                        900
```

FIGURE I   b


LeuLeuTrpLysIleAlaArgAlaGlyAlaArgSerAlaValGlyThrGluAlaArgArg
TTGTTGTGGAAAATCGCTCGCGCCGGCGCCCGTTCCGCAGTGGGCACCGAGGCGCGTCGC

GlnPheArgTyrAspGlyAspMetAsnIleGlyValIleThrAspPheGluLeuGluVal
CAGTTCCGCTACGACGGCGACATGAATATCGGCGTGATCACCGATTTCGAGCTGGAAGTG
                                                        1000
ArgAsnAlaLeuAsnArgArgAlaHisAlaValGlyAlaGlnAspValValGlnHisGly
CGCAATGCGCTGAACAGGCGGGCGCACGCCGTCGGCGCGCAGGACGTGGTCCAGCATGGC

ThrGluGlnAsnAsnProPheProGluAlaAspGluLysIlePheValValSerAlaThr
ACTGAGCAGAACAATCCTTTCCCGGAGGCAGATGAGAAGATTTTCGTCGTATCGGCCACC
              1100
GlyGluSerGlnMetLeuThrArgGlyGlnLeuLysGluTyrIleGlyGlnGlnArgGly
GGTGAAAGCCAGATGCTCACGCGCGGGCAACTGAAGGAATACATTGGCCAGCAGCGCGGC
                                                        1200
GluGlyTyrValPheTyrGluAsnArgAlaTyrGlyValAlaGlyLysSerLeuPheAsp
GAGGGCTATGTCTTCTACGAGAACCGTGCATACGGCGTGGCGGGGAAAAGCCTGTTCGAC

AspGlyLeuGlyAlaAlaProGlyValProSerGlyArgSerLysPheSerProAspVal
GATGGGCTGGGAGCCGCGCCCGGCGTGCCGAGCGGACGTTCGAAGTTCTCGCCGGATGTA
                                                        1300
LeuGluThrValProAlaSerProGlyLeuArgArgProSerLeuGlyAlaValGluArg
CTGGAAACGGTGCCGGCGTCACCCGGATTGCGGCGGCCGTCGCTGGGCGCAGTGGAACGC

GlnAspSerGlyTyrAspSerLeuAspGlyValGlySerArgSerPheSerLeuGlyGlu
CAGGATTCCGGCTATGACAGCCTTGATGGGGTGGGATCGCGATCGTTCTCGTTGGGCGAG
              1400
ValSerAspMetAlaAlaValGluAlaAlaGluLeuGluMetThrArgGlnValLeuHis
GTGTCCGACATGGCCGCCGTGGAAGCGGCGGAACTGGAAATGACCCGGCAAGTCTTGCAC
                                                        1500
AlaGlyAlaArgGlnAspAspAlaGluProGlyValSerGlyAlaSerAlaHisTrpGly
GCCGGGGCGCGGCAGGACGATGCCGAGCCGGGCGTGAGCGGTGCGTCGGCGCACTGGGGG

GlnArgAlaLeuGlnGlyAlaGlnAlaValAlaAlaAlaGlnArgLeuValHisAlaIle
CAGCGGGCGCTGCAGGGCGCCCAGGCGGTGGCGGCGGCGCAGCGGCTGGTTCATGCCATT
                                                        1600
AlaLeuMetThrGlnPheGlyArgAlaGlySerThrAsnThrProGlnGluAlaAlaSer
GCCCTGATGACGCAATTCGGCCGGGCCGGTTCCACCAACACGCCGCAGGAAGCGGCCTCG

LeuSerAlaAlaValPheGlyLeuGlyGluAlaSerSerAlaValAlaGluThrValSer
TTGTCGGCGGCCGTGTTCGGCTTGGGCGAGGCCAGCAGCGCCGTGGCCGAAACCGTGAGC
              1700
GlyPhePheArgGlySerSerArgTrpAlaGlyGlyPheGlyValAlaGlyGlyAlaMet
GGTTTTTTCCGCGGGTCTTCGCGCTGGGCCGGCGGTTTCGGCGTGGCTGGCGGCGCGATG
                                                        1800

FIGURE I c

AlaLeuGlyGlyGlyIleAlaAlaAlaValGlyAlaGlyMetSerLeuThrAspAspAla
GCGCTGGGAGGCGGCATCGCCGCGGCCGTTGGCGCCGGGATGTCGTTGACCGATGACGCG

ProAlaGlyGlnLysAlaAlaAlaGlyAlaGluIleAlaLeuGlnLeuThrGlyGlyThr
CCGGCCGGACAGAAGGCCGCCGCCGGCGCCGAGATCGCGCTGCAGTTGACAGGTGGAACG
1900

ValGluLeuAlaSerSerIleAlaLeuAlaLeuAlaAlaAlaArgGlyValThrSerGly
GTCGAGCTGGCTTCTTCCATCGCGTTGGCGCTGGCCGCGGCGCGCGGCGTGACCAGCGGC

LeuGlnValAlaGlyAlaSerAlaGlyAlaAlaAlaGlyAlaLeuAlaAlaAlaLeuSer
TTGCAGGTGGCCGGGGCGTCGGCCGGGGCGGCTGCCGGCGCATTGGCCGCGGCGCTCAGT
2000

ProMetGluIleTyrGlyLeuValGlnGlnSerHisTyrAlaAspGlnLeuAspLysLeu
CCCATGGAGATCTACGGCCTGGTGCAGCAATCGCACTATGCGGATCAGCTGGACAAGCTG
2100

AlaGlnGluSerSerAlaTyrGlyTyrGluGlyAspAlaLeuLeuAlaGlnLeuTyrArg
GCGCAGGAATCGAGCGCATACGGTTACGAGGGCGACGCCTTGCTGGCCCAGCTGTATCGC

AspLysThrAlaAlaGluGlyAlaValAlaGlyValSerAlaValLeuSerThrValGly
GACAAGACGGCCGCCGAGGGCGCCGTCGCCGGCGTCTCCGCCGTCCTGAGCACGGTGGGG
2200

AlaAlaValSerIleAlaAlaAlaAlaSerValValGlyAlaProValAlaValValThr
GCGGCGGTGTCGATCGCCGCGGCGGCCAGCGTGGTAGGGGCCCCGGTGGCGGTGGTCACT

SerLeuLeuThrGlyAlaLeuAsnGlyIleLeuArgGlyValGlnGlnProIleIleGlu
TCCTTGCTGACCGGGGCTCTCAACGGCATCCTGCGCGGCGTGCAGCAGCCCATCATCGAA
2300

LysLeuAlaAsnAspTyrAlaArgLysIleAspGluLeuGlyGlyProGlnAlaTyrPhe
AAGCTGGCCAACGATTACGCTCGCAAGATCGACGAGCTGGGCGGGCCGCAAGCGTACTTC
2400

GluLysAsnLeuGlnAlaArgHisGluGlnLeuAlaAsnSerAspGlyLeuArgLysMet
GAGAAAAACCTGCAGGCGCGTCACGAACAACTGGCCAATTCGGACGGCCTACGGAAAATG

LeuAlaAspLeuGlnAlaGlyTrpAsnAlaSerSerValIleGlyValGlnThrThrGlu
CTGGCCGACCTGCAGGCCGGTTGGAACGCCAGCAGCGTGATCGGGGTGCAGACGACAGAG
2500

IleSerLysSerAlaLeuGluLeuAlaAlaIleThrGlyAsnAlaAspAsnLeuLysSer
ATCTCCAAGTCGGCGCTCGAACTGGCCGCCATTACCGGCAACGCGGACAACCTGAAATCC

ValAspValPheValAspArgPheValGlnGlyGluArgValAlaGlyGlnProValVal
GTCGACGTGTTCGTGGACCGCTTCGTCCAGGGCGAGCGGGTGGCCGGCCAGCCGGTGGTC
2600

LeuAspValAlaAlaGlyGlyIleAspIleAlaSerArgLysGlyGluArgProAlaLeu
CTCGACGTCGCCGCCGGCGGCATCGATATCGCCAGCCGCAAGGGCGAGCGGCCGGCGCTG
EcoRV
2700

FIGURE I d

```
ThrPheIleThrProLeuAlaAlaProGlyGluGluGlnArgArgArgThrLysThrGly
ACGTTCATCACGCCGCTGGCCGCGCCAGGAGAAGAGCAGCGCCGGCGCACGAAAACGGGC

LysSerGluPheThrThrPheValGluIleValGlyLysGlnAspArgTrpArgIleArg
AAGAGCGAATTCACCACATTCGTCGAGATCGTGGGCAAGCAGGACCGCTGGCGCATCCGG
     EcoRI                                    2800
AspGlyAlaAlaAspThrThrIleAspLeuAlaLysValValSerGlnLeuValAspAla
GACGGCGCGGCCGACACCACCATCGATCTGGCCAAGGTGGTGTCGCAACTGGTCGACGCC

AsnGlyValLeuLysHisSerIleLysLeuAspValIleGlyGlyAspGlyAspAspVal
AATGGCGTGCTCAAGCACAGCATCAAACTGGATGTGATCGGCGGAGATGGCGATGACGTC
     2900
ValLeuAlaAsnAlaSerArgIleHisTyrAspGlyGlyAlaGlyThrAsnThrValSer
GTGCTTGCCAATGCTTCGCGCATCCATTATGACGGCGGCGCGGGCACCAACACGGTCAGC
                                                        3000
TyrAlaAlaLeuGlyArgGlnAspSerIleThrValSerAlaAspGlyGluArgPheAsn
TATGCCGCCCTGGGTCGACAGGATTCCATTACCGTGTCCGCCGACGGGAACGTTTCAAC

ValArgLysGlnLeuAsnAsnAlaAsnValTyrArgGluGlyValAlaThrGlnThrThr
GTGCGCAAGCAGTTGAACAACGCCAACGTGTATCGCGAAGGCGTGGCTACCCAGACAACC
                                              3100
AlaTyrGlyLysArgThrGluAsnValGlnTyrArgHisValGluLeuAlaArgValGly
GCCTACGGCAAGCGCACGGAGAATGTCCAATACCGCCATGTCGAGCTGGCCCGTGTCGGG

GlnValValGluValAspThrLeuGluHisValGlnHisIleIleGlyGlyAlaGlyAsn
CAAGTGGTGGAGGTCGACACGCTCGAGCATGTGCAGCACATCATCGGCGGGGCCGGCAAC
     3200
AspSerIleThrGlyAsnAlaHisAspAsnPheLeuAlaGlyGlySerGlyAspAspArg
GATTCGATCACCGGCAATGCGCACGACAACTTCCTAGCCGGCGGGTCGGGCGACGACAGG
                                                        3300
LeuAspGlyGlyAlaGlyAsnAspThrLeuValGlyGlyGluGlyGlnAsnThrValIle
CTGGATGGCGGCGCCGGCAACGACACCCTGGTTGGCGGCGAGGGCCAAAACACGGTCATC

GlyGlyAlaGlyAspAspValPheLeuGlnAspLeuGlyValTrpSerAsnGlnLeuAsp
GGCGGCGCCGGCGACGACGTATTCCTGCAGGACCTGGGGGGTATGGAGCAACCAGCTCGAT
                                              3400
GlyGlyAlaGlyValAspThrValLysTyrAsnValHisGlnProSerGluGluArgLeu
GGCGGCGCGGGCGTCGATACCGTGAAGTACAACGTGCACCAGCCTTCCGAGGAGCGCCTC

GluArgMetGlyAspThrGluIleHisAlaAspLeuGlnLysGlyThrValGluLysTrp
GAACGCATGGGCGACACGGGCATCCATGCCGATCTTCAAAAGGGCACGGTCGAGAAGTGG
     3500
ProAlaLeuAsnLeuPheSerValAspHisValLysAsnIleGluAsnLeuHisGlySer
CCGGCCCTGAACCTGTTCAGCGTCGACCATGTCAAGAATATCGAGAATCTGCACGGCTCC
                                                        3600
```

## FIGURE I e

```
ArgLeuAsnAspArgIleAlaGlyAspAspGlnAspAsnGluLeuTrpGlyHisAspGly
CGCCTAAACGACCGCATCGCCGGCGACGACCAGGACAACGAGCTCTGGGGCCACGATGGC
                                                  SacI

AsnAspThrIleArgGluArgGluGlyAspAspIleLeuArgGluGlyLeuGlyLeuAsp
AACGACACGATACGCGGCCGGGGCGGCGACGACATCCTGCGCGGCGGCCTGGGCCTGGAC
                                        3700

ThrLeuTyrGlyGluAspGlyAsnAspIlePheLeuGlnAspAspGluThrValSerAsp
ACGCTGTATGGCGAGGACGGCAACGACATCTTCCTGCAGGACGACGAGACCGTCAGCGAT

AspIleAspGlyGlyAlaGlyLeuAspThrValAspTyrSerAlaMetIleHisProGly
GACATCGACGGCGGCGCGGGGCTGGACACCGTCGACTACTCCGCCATGATCCATCCAGGC
          3800

ArgIleValAlaProHisGluTyrGlyPheGlyIleGluAlaAspLeuSerArgGluTrp
AGGATCGTTGCGCCGCATGAATACGGCTTCGGGATCGAGGCGGACCTGTCCAGGGAATGG
                                                          3900

ValArgLysAlaSerAlaLeuGlyValAspTyrTyrAspAsnValArgAsnValGluAsn
GTGCGCAAGGCGTCCGCGCTGGGCGTGGACTATTACGATAATGTCCGCAATGTCGAAAAC

ValIleGlyThrSerMetLysAspValLeuIleGlyAspAlaGlnAlaAsnThrLeuMet
GTCATCGGTACGAGCATGAAGGATGTGCTCATCGGCGACGCGCAAGCCAATACCCTGATG
                                        4000

GlyGlnGlyGlyAspAspThrValArgGlyGlyAspGlyAspAspLeuLeuPheGlyGly
GGCCAGGGCGGCGACGATACCGTGCGCGGCGGCGACGGCGATGATCTGCTGTTCGGCGGC

AspGluAsnAspMetLeuTyrGlyAspAlaGlyAsnAspThrLeuTyrGlyGluLeuGlu
GACGGCAACGACATGCTGTATGGCGACGCCGGCAACGACACCCTCTACGGGGGGCTGGGC
          4100

AspAspThrLeuGluGluGlyAlaGlyAsnAspTrpPheGlyGlnThrGlnAlaArgGlu
GACGATACCCTTGAAGGCGGCGCGGGCAACGATTGGTTCGGCCAGACGCAGGCGCGCGAG
                                                  4200

HisAspValLeuArgGlyGlyAspGlyValAspThrValAspTyrSerGlnThrGlyAla
CATGACGTGCTGCGCGGCGGAGATGGGGTGGATACCGTCGATTACAGCCAGACCGGCGCG

HisAlaGlyIleAlaAlaGlyArgIleGlyLeuGlyIleLeuAlaAspLeuGlyAlaGly
CATGCCGGCATTGCCGCGGGTCGCATCGGGCTGGGCATCCTGGCTGACCTGGGCGCCGGC
                                        4300

ArgValAspLysLeuGlyGluAlaGlySerSerAlaTyrAspThrValSerGlyIleGlu
CGCGTCGACAAGCTGGGCGAGGCCGGCAGCAGCGCCTACGATACGGTTTCCGGTATCGAG

AsnValValGlyThrGluLeuAlaAspArgIleArgAlaMetArgArgProThrCysCys
AACGTGGTGGGCACGGAACTGGCCGACCGCATCCGGGCGATGCGCAGGCCAACGTGCTGC
          4400

AlaAlaArgValAlaProThrCysLeuArgAlaAlaArgAlaThrMetCysCysTrpAla
GCGGCGCGGGTGGCGCCGACGTGCTTGCGGGCGGCGAGGGCGACGATGTGCTGCTGGGCG
                                                  4500
```

AlaThrAlaThrThrSerCysArgAlaThrProAspAlaIleAlaCysThrAlaLysPro
GCGACGGCGACGACCAGCTGTCGGGCGACGCCGGACGCGATCGCTTGTACGGCGAAGCCG

ValThrThrGlySerSerArgMetProProMetProAlaAsnLeuLeuAspGlyGlyAsp
GTGACGACTGGTTCTTCCAGGATGCCGCCAATGCCGGCGAATCTGCTCGACGGCGGCGAC
4600

GluArgAspThrValAspGlnArgPro
GGCCGCGATACCGTGGATCAGCGCCCGGG
SmaI

FIGURE I f

## FIGURE 2

```
       10        20        30        40        50
MTRNKFIPNKFSIISFSVLLFAISSSQAIEVNAHNEHYTESDIKRNHKTEKNKTEKEKFK

       70        80        90       100       110
DSINNLVKTEFTNETLDKIQQTQDLLKKIPKDVLEIYSELGGEIYFTDIDLVEHKELQDL

      130       140       150       160       170
SEEEKNSHNSRGEKVPFASRFVFEKKRETPKLIINIKDYAINSEQSKEVYYEIGKGISLD

      190       200       210       220       230
IISKDKSLDPEFLNLIKSLSDDSDSSDLLFSQKFKEKLELNNKSIDINFIKENLTEFQHA

      250       260       270       280       290
FSLAFSYYFAPDHRTVLELYAPDMFEYHNKLEKGGFEKISESLKKEGVEKDRIDVLKGEK
                                         +    +    ++
                                         HQQSHQAGYANA
                                                   10

      310       320       330       340       350
 ALKASGLVPEHADAFKKIARELNTYILFRPVNKLATNLIKSGVATKGLNVHGKSSDKGPV
+ +   ++  +   +  +  + +*+  *+ ++** **   +* **  ****** **+****
 ADRESGIPAAVLDGIKAVAKEKNATLMFRLVNPHSTSLIAEGVATKGLGVHAKSSDWGLQ
    20        30        40        50        60        70

      370       380       390       400       410
AGYIPFDQDLSKKHGQQLAVEKGNLENKKSITEHEGEIGKIPLKLDHLRIEELKENGIIL
***** + +***  *      *     ++  *  * *+*+ *  +++ *++ *++
AGYIPVNPNLSKLFGRAPEVIARADHDVNSSLAH-GHTA-VDLTLSKERLDYLRQAGLVT
    80        90       100       110       120

      430       440       450       460       470
KGKKEIDNGKKYYLLESNHQVYEFRISDENNE---VQYKTKEGKITVLGEKFNWRNIEVH
 *  +  +        *   +****+ + +  ***+ *     *+  *     * +
-GMADGVVASNHAGYEQ----FEFRVKETSDGRYAVQYRRKG------GDDF-----EAV
            140                150       160          170

 480       490       500       510               520
AKNVEGVLKPLTADYDLFALAPSLTEIKKQIPQKEWD-------KVVNTPNSLEKQKGV-
  ++   ***** *+**+ *  *++ +              +  *    *+
KVIGNAAGIPLTADIDMFAIMPHLSNFRDSARSSVTSGDSVTDILARTRRAASEATGGLD
  180       190       200       210       220       230

      530       540       550       560       570
---TNLLIKY--GIE-RKPDSTKGTLSHWQKQM---LDRLNEAV-KYTGYTGGDVVNHGT
   .** *    *  *  *+  * *  +    + ++ **+*** *
RERIDLLNKIARGTEARRQFRYDGDM-NIGVITDFELEVRNALNRRAHAVGAQDVVQHGT
 240       250       260       270       280       290

580       590        600       610       620       630
EQDNEEPPEKDNEIFIINPEGEF-ILTKNWEHTGRFIEKNITGKDYLYYFNRSYNKIAPG
**+* *** **+ **+++ +  ** +***+  ++   +* + *+*+ +*+* +* *
EQNNP-PPEADEKIFVVSATGESQMLTRG-QLKE-YIGQQR-GEGYVFYENRAY-GVA-G
      310       320       330       348       350

640         650       660       670       680       690
NKAYIE---WTDP-ITKAKINTIPTSAEFIKNLSSIRRSSHVGVYK-DSGDKDEFAKKES
 *+ +     +  * ++ ++    *   * +++**++ +* * ****       *
-KSLFDDGLGAAPGVPSGRSKFSPDVLETVPASPGLRRPSLGAVERQDSG-YDSLDGVGS
     360       370       380       390       400       410

      700       710       720       730       740       750
VKKIAGYLSDYYNSANHIFSQEKKRKISIFRGIQAYNEIENVLKSKQIAPEYKNYFQYLK
 *  +**       *     +   + + +* + *+   *          +
RSFSLGEVSDHAAVEAAELEHTRQVLHAGARQDDAEPGVSGASAHHGQRALQGAQAVAAA
     420       430       440       450       460       470

      760       770       780       790
ERITNQVQLLLTHQKSNIEFKLLYKQLNFTENETDHFEVFQKIIDEK
+*+        *+    ++                +     +
QRLVHAIALMTQFGRAGSTNTRQEAASLSAAVFGLGEASSAVAETVSGFFRGSSRWAGGF
     480       490       500       510       520       530
```

TGCGATCATTCGGCATGTACGGTCCAGCTGCGCGCGAGCGGCGGCCGCGTCCAGCGCGCGGCCTCGGTACTCCTTGACGCGCGCGGTGTCGCCGCCGCGCCGAACGCGCAGCGAACGGCC

CACGCTGTCGGGGTGCCGTTCGGCCAGCGCGCGGCGCAGCGCACGATTGTCGTCGCGCGAGAATGGCGCGATCCAGTCGATGATCCACAGTCGGTCGCCGCAGTTCCAGGCATTCCCGCC

CAGCGACGAGGGCGCCATGACATAGGAGAGTTCGGTGTCGGCGTCCATTAGGGCCCAGCTGCAGTATGCAACCGGCACGTCATTGCATCGCAGCAGAATGTATTGGCCCAGTTGAATCGG

CGCGAGCGCTGTTGCGTGCCGAGCAGATGCACCGGCCAGTCGCGGTGCATGGGAGAGTTCATCCACAGCCAGGCAATATTGCCCAGTGCCGCGAAGTCGTCGGTGGGATTGAGGAGGGAGG

GCGCTTGGGCGGACGGAAGCATGACATCGGTGCATGGTGGAGCGGGGGGGCATATTCCGTGTTGGGTGCGCGCATGGCAAGCCGCCGGCGCATCATGGTTGCGCCGGAATGGCTTTTCTTA

CATGTTTCCAGGATATGTCCGTATTTCGGGCGATGCCTCGGTCGCGGCGCCTGCTTTTGTCGAACATGTGCAATGTTGTTGTCGCGATCGCGTTGGCGCTTGCTCGCTTATTTATCTCCC

TTGAAGCCTTGTTCTTCTTTTTCATTAGAAAGAAATATGCGCTTTGTGTTTAGGATGATTTTCCTGTCCGAGTAGGGTGGATCCAAATTTTTCCGGATTGGTGGGAATTTGTGCATTTTCAC

EP 0 366 549 A1

FIGURE 3 A

EP 0 366 549 A1

TGCGAATGTTGGAATAATTTCGCCCATCGTCATACGACATGCTGGATGTTTGGTTCTTGCAGAAGGATGAGGTTCTGAGCGCTACACACCGGTTGCGTCGGTGCGAATCCGTTCAATCGA

```
        M  Q  Q  S  H  Q  A  G  Y  A  N  A  A  D  R  E  S  G  I  P  A  A  V  L  D  G  I  K  A  V  A  K  E  K
```
CTACTTATCGACAGATCCACATGCAGCAATCGCATCAGGCTGGTTACGCAAACGCCGCCGACCGGGAGTCTGGCATCCCCGCAGCCGTACTCGATGGCATCAAGGCCGTGGCGAAGGAAA

```
N  A  T  L  M  F  R  L  V  N  P  H  S  T  S  L  I  A  E  G  V  A  T  K  G  L  G  V  H  A  K  S  S  D  W  G  L  Q  A  G
```
AAAACGCCACATTGATGTTCCGCCTGGTCAACCCCCATTCCACCAGCCTGATTGCCGAAGGGGTGGCCACCAAAGGATTGGGCGTGCACGCCAAGTCGTCCGATTGGGGGGTTGCAGGCGG

```
Y  I  P  V  N  P  N  L  S  K  L  F  G  R  A  P  E  V  I  A  R  A  D  N  D  V  N  S  S  L  A  H  G  H  T  A  V  D  L  T
```
GCTACATTCCCGTCAACCCGAATCTTTCCAAACTGTTCGGCCGTGCGCCCGAGGTGATCGCGCGGGCCGACAACGACGTCAACAGCAGCCTGGCGCATGGCCATACCGCGGTCGACCTGA

```
L  S  K  E  R  L  D  Y  L  R  Q  A  G  L  V  T  G  M  A  D  G  V  V  A  S  N  H  A  G  Y  E  Q  F  E  F  R  V  K  E  T
```
CGCTGTCGAAAGAGCGGCTTGACTATCTGCGGCAAGCGGGCCTGGTCACCGGCATGGCCGATGGCGTGGTCGCGAGCAACCACGCAGGCTACGAGCAGTTCGAGTTTCGCGTGAAGGAAA

```
S  D  G  R  Y  A  V  Q  Y  R  R  K  G  G  D  D  F  E  A  V  K  V  I  G  N  A  A  G  I  P  L  T  A  D  I  D  M  F  A  I
```
CCTCGGACGGGCGCTATGCCGTGCAGTATCGCCGCAAGGGCGGCGACGATTTCGAGGCGGTCAAGGTGATCGGCAATGCCGCCGGTATTCCACTGACGGCGGATATCGACATGTTCGCCA

FIGURE 3 B

```
M  P  H  L  S  N  F  R  D  S  A  R  S  S  V  T  S  G  D  S  V  T  D  Y  L  A  R  T  R  R  A  A  S  E  A  T  G  G  L  D
TTATGCCGCATCTGTCCAACTTCCGCGACTCGGCGCGCAGTTCGGTGACCAGCGGCGATTCGGTGACCGATTACCTGGCGCGCACGCGGCGGGCCGCCAGCGAGGCCACGGGCGGCCTGG
     1570      1580      1590      1600      1610      1620      1630      1640      1650      1660      1670      1680


R  E  R  I  D  L  L  W  K  I  A  R  A  G  A  R  S  A  V  G  T  E  A  R  R  Q  F  R  Y  D  G  D  M  N  I  G  V  I  T  D
ATCGCGAACGCATCGACTTGTTGTGGAAAATCGCTCGCGCCGGCGCCCGTTCCGCAGTGGGCACCGAGGCGCGTCGCCAGTTCCGCTACGACGGCGACATGAATATCGGCGTGATCACCG
     1690      1700      1710      1720      1730      1740      1750      1760      1770      1780      1790      1800


F  E  L  E  V  R  N  A  L  N  R  R  A  H  A  V  G  A  Q  D  V  V  Q  H  G  T  E  Q  N  N  P  F  P  E  A  D  E  K  I  F
ATTTCGAGCTGGAAGTGCGCAATGCGCTGAACAGGCGGGCGCACGCCGTCGGCGCGCAGGACGTGGTCCAGCATGGCACTGAGCAGAACAATCCTTTCCCGGAGGCAGATGAGAAGATTT
     1810      1820      1830      1840      1850      1860      1870      1880      1890      1900      1910      1920


V  V  S  A  T  G  E  S  Q  M  L  T  R  G  Q  L  K  E  Y  I  G  Q  Q  R  G  E  G  Y  V  F  Y  E  N  R  A  Y  G  V  A  G
TCGTCGTATCGGCCACCGGTGAAAGCCAGATGCTCACGCGCGGGCAACTGAAGGAATACATTGGCCAGCAGCGCGGCGAGGGCTATGTCTTCTACGAGAACCGTGCATACGGCGTGGCGG
     1930      1940      1950      1960      1970      1980      1990      2000      2010      2020      2030      2040


K  S  L  F  D  D  G  L  G  A  A  P  G  V  P  S  G  R  S  K  F  S  P  D  V  L  E  T  V  P  A  S  P  G  L  R  R  P  S  L
GGAAAAGCCTGTTCGACGATGGGCTGGGAGCCGCGCCCGGCGTGCCGAGCGGACGTTCGAAGTTCTCGCCGGATGTACTGGAAACGGTGCCGGCGTCACCCGGATTGCGGCGGCCGTCGC
     2050      2060      2070      2080      2090      2100      2110      2120      2130      2140      2150      2160
```

EP 0 366 549 A1

FIGURE 3 C

G A V E R Q D S G Y D S L D G V G S R S F S L G E V S D M A A V E A A E L E M T
TGGGCGCAGTGGAACGCCAGGATTCCGGCTATGACAGCCTTGATGGGGTGGGATCGCGATCGTTCTCGTTGGGCGAGGTGTCCGACATGGCCGCCGTGGAAGCGGCGGAACTGGAAATGA

R Q V L H A G A R Q D D A E P G V S G A S A H W G Q R A L Q G A Q A V A A A Q R
CCCGGCAAGTCTTGCACGCCGGGGCGCGGCAGGACGATGCCGAGCCGGGCGTGAGCGGTGCGTCGGCGCACTGGGGGCAGCGGGCGCTGCAGGGCGCCCAGGCGGTGGCGGCGGCGCAGC

L V H A I A L M T Q F G R A G S T N T P Q E A A S L S A A V F G L G E A S S A V
GGCTGGTTCATGCCATTGCCCTGATGACGCAATTCGGCCGGGGCCGGTTCCACCAACACGCCGCAGGAAGCGGCCTCGTTGTCGGCGGCCGTGTTCGGCTTGGGCGAGGCCAGCAGCGCCG

A E T V S G F F R G S S R W A G G F G V A G G A M A L G G G I A A A V G A G M S
TGGCCGAAACCGTGAGCGGTTTTTTTCCGCGGGTCTTCGCGCTGGGCCGGCGGTTTCGGCGTGGCTGGCGGCGCGATGGCGCTGGGAGGCGGCATCGCCGCGGCCGTTGGCGCCGGGATGT

L T D D A P A G Q K A A A G A E I A L Q L T G G T V E L A S S I A L A L A A A R
CGTTGACCGATGACGCGCCGGCCGGACAGAAGGCCGCCGCCGGCGCCGAGATCGCGCTGCAGTTGACAGGTGGAACGGTCGAGCTGGCTTCTTCCATCGCGTTGGCGCTGGCCGCGGCGC

EP 0 366 549 A1

FIGURE 3 D

EP 0 366 549 A1

```
  G  V  T  S  G  L  Q  V  A  G  A  S  A  G  A  A  A  G  A  L  A  A  A  L  S  P  M  E  I  Y  G  L  V  Q  Q  S  H  Y  A  D
GCGGCGTGACCAGCGGCTTGCAGGTGGCCGGGGCGTCGGCCGGGGCGGCTGCCGGCGCATTGGCCGCGGCGCTCAGTCCCATGGAGATCTACGGCCTGGTGCAGCAATCGCACTATGCGG
.     2770      2780      2790      2800      2810      2820      2830      2840      2850      2860      2870      2880


  Q  L  D  K  L  A  Q  E  S  S  A  Y  G  Y  E  G  D  A  L  L  A  Q  L  Y  R  D  K  T  A  A  E  G  A  V  A  G  V  S  A  V
ATCAGCTGGACAAGCTGGCGCAGGAATCGAGCGCATACGGTTACGAGGGCGACGCCTTGCTGGCCCAGCTGTATCGCGACAAGACGGCCGCCGAGGGCGCCGTCGCCGGCGTCTCCGCCG
.     2890      2900      2910      2920      2930      2940      2950      2960      2970      2980      2990      3000


  L  S  T  V  G  A  A  V  S  I  A  A  A  A  S  V  V  G  A  P  V  A  V  V  T  S  L  L  T  G  A  L  N  G  I  L  R  G  V  Q
TCCTGAGCACGGTGGGGGCGGCGGTGTCGATCGCCGCGGCGGCCAGCGTGGTAGGGGCCCCGGTGGCGGTGGTCACTTCCTTGCTGACCGGGGGCTCTCAACGGCATCCTGCGCGGCGTGC
.     3010      3020      3030      3040      3050      3060      3070      3080      3090      3100      3110      3120


  Q  P  I  I  E  K  L  A  N  D  Y  A  R  K  I  D  E  L  G  G  P  Q  A  Y  F  E  K  N  L  Q  A  R  H  E  Q  L  A  N  S  D
AGCAGCCCATCATCGAAAAGCTGGCCAACGATTACGCTCGCAAGATCGACGAGCTGGGCGGGGCCGCAAGCGTACTTCGAGAAAAAACCTGCAGGCGCGTCACGAACAACTGGCCAATTCGG
.     3130      3140      3150      3160      3170      3180      3190      3200      3210      3220      3230      3240


  G  L  R  K  M  L  A  D  L  Q  A  G  W  N  A  S  S  V  I  G  V  Q  T  T  E  I  S  K  S  A  L  E  L  A  A  I  T  G  N  A
ACGGCCTACGGAAAAATGCTGGCCGACCTGCAGGCCGGTTGGAACGCCAGCAGCGTGATCGGGGTGCAGACGACAGAGATCTCCAAGTCGGCGCTCGAACTGGCCGCCATTACCGGCAACG
.     3250      3260      3270      3280      3290      3300      3310      3320      3330      3340      3350      3360
```

FIGURE 3 E

D N L K S V D V F V D R F V Q G E R V A G Q P V V L D V A A G G I D I A S R K G
CGGACAACCTGAAATCCGTCGACGTGTTCGTGGACCGCTTCGTCCAGGGCGAGCGGGTGGCCGGCCAGCCGGTGGTCCTCGACGTCGCCGCCGGCGGCATCGATATCGCCAGCCGCAAGG

E R P A L T F I T P L A A P G E E Q R R R T K T G K S E F T T F V E I V G K Q D
GCGAGCGGCCGGCGCTGACGTTCATCACGCCGCTGGCCGCGCCAGGAGAAGAGCAGCGCCGGCGCACGAAAACGGGCAAGAGCGAATTCACCACATTCGTCGAGATCGTGGGCAAGCAGG

R W R I R D G A A D T T I D L A K V V S Q L V D A N G V L K H S I K L D V I G G
ACCGCTGGCGCATCCGGGACGGCGCGGCCGACACCACCATCGATCTGGCCAAGGTGGTGTCGCAACTGGTCGACGCCAATGGCGTGCTCAAGCACAGCATCAAACTGGATGTGATCGGCG

D G D D V V L A N A S R I H Y D G G A G T N T V S Y A A L G R Q D S I T V S A D
GAGATGGCGATGACGTCGTGCTTGCCAATGCTTCGCGCATCCATTATGACGGCGGCGCGGGCACCAACACGGTCAGCTATGCCGCCCTGGGTCGACAGGATTCCATTACCGTGTCCGCCG

G E R F N V R K Q L N N A N V Y R E G V A T Q T T A Y G K R T E N V Q Y R H V E
ACGGGGAACGTTTCAACGTGCGCAAGCAGTTGAACAACGCCAACGTGTATCGCGAAGGCGTGGCTACCCAGACAACCGCCTACGGCAAGCGCACGGAGAATGTCCAATACCGCCATGTCG

EP 0 366 549 A1

FIGURE 3 F

EP 0 366 549 A1

```
L A R V G Q V V E V D T L E H V Q H I I G G A G N D S I T G N A H D N F L A G G
AGCTGGCCCGTGTCGGGCAAGTGGTGGAGGTCGACACGCTCGAGCATGTGCAGCACATCATCGGCGGGGCCGGCAACGATTCGATCACCGGCAATGCGCACGACAACTTCCTAGCCGGCG
     3970      3980      3990      4000      4010      4020      4030      4040      4050      4060      4070      4080


S G D D R L D G G A G N D T L V G G E G Q N T V I G G A G D D V F L Q D L G V W
GGTCGGGCGACGACAGGCTGGATGGCGGCGCCGGCAACGACACCCTGGTTGGCGGCGAGGGCCAAAACACGGTCATCGGCGGCGCCGGCGACGACGTATTCCTGCAGGACCTGGGGGGTAT
     4090      4100      4110      4120      4130      4140      4150      4160      4170      4180      4190      4200


S N Q L D G G A G V D T V K Y N V H Q P S E E R L E R M G D T G I H A D L Q K G
GGAGCAACCAGCTCGATGGCGGCGCGGGCGTCGATACCGTGAAGTACAACGTGCACCAGCCTTCCGAGGAGCGCCTCGAACGCATGGGCGACACGGGCATCCATGCCGATCTTCAAAAGG
     4210 ·    4220      4230      4240      4250      4260      4270      4280      4290      4300      4310      4320


T V E K W P A L N L F S V D H V K N I E N L H G S R L N D R I A G D D Q D N E L
GCACGGTCGAGAAGTGGCCGGCCCTGAACCTGTTCAGCGTCGACCATGTCAAGAATATCGAGAATCTGCACGGCTCCCGCCTAAACGACCGCATCGCCGGCGACGACCAGGACAACGAGC
     4330      4340      4350      4360      4370      4380      4390      4400      4410      4420      4430      4440


W G H D G N D T I R G R G G D D I L R G G L G L D T L Y G E D G N D I F L Q D D
TCTGGGGCCACGATGGCAACGACACGATACGCGGCCGGGGCGGCGACGACATCCTGCGCGGCGGCCTGGGCCTGGACACGCTGTATGGCGAGGACGGCAACGACATCTTCCTGCAGGACG
     4450      4460      4470      4480      4490      4500      4510      4520      4530      4540      4550      4560
```

FIGURE 3 G

E T V S D D I D G G A G L D T V D Y S A M I H P G R I V A P H E Y G F G I E A D
ACGAGACCGTCAGCGATGACATCGACGGCGGCGCGGGGGCTGGACACCGTCGACTACTCCGCCATGATCCATCCAGGCAGGATCGTTGCGCCGCATGAATACGGCTTCGGGATCGAGGCGG

L S R E W V R K A S A L G V D Y Y D N V R N V E N V I G T S M K D V L I G D A Q
ACCTGTCCAGGGAATGGGTGCGCAAGGCGTCCGCGCTGGGCGTGGACTATTACGATAATGTCCGCAATGTCGAAAACGTCATCGGTACGAGCATGAAGGATGTGCTCATCGGCGACGCGC

A N T L M G Q G G D D T V R G G D G D D L L F G G D G N D M L Y G D A G N D T L
AAGCCAATACCCTGATGGGCCAGGGCGGCGACGATACCGTGCGCGGCGGCGACGGCGATGATCTGCTGTTCGGCGGCGACGGCAACGACATGCTGTATGGCGACGCCGGCAACGACACCC

Y G G L G D D T L E G G A G N D W F G Q T Q A R E H D V L R G G D G V D T V D Y
TCTACGGGGGGCTGGGCGACGATACCCTTGAAGGCGGCGCGGGCAACGATTGGTTCGGCCAGACGCAGGCGCGCGAGCATGACGTGCTGCGCGGCGGAGATGGGGTGGATACCGTCGATT

S Q T G A H A G I A A G R I G L G I L A D L G A G R V D K L G E A G S S A Y D T
ACAGCCAGACCGGCGCGCATGCCGGCATTGCCGCGGGTCGCATCGGGCTGGGCATCCTGGCTGACCTGGGCGCCGGCCGCGTCGACAAGCTGGGCGAGGCCGGCAGCAGCGCCTACGATA

EP 0 366 549 A1

FIGURE 3 H

V S G I E N V V G T E L A D R I T G D A Q A N V L R G A G G A D V L A G G E G D
CGGTTTCCGGTATCGAGAACGTGGTGGGCACGGAACTGGCCGACCGCATCACGGGCGATGCGCAGGCCAACGTGCTGCGCGGCGCGGGTGGCGCCGACGTGCTTGCGGGCGGCGAGGGCG

D V L L G G D G D D Q L S G D A G R D R L Y G E A G D D W F F Q D A A N A G N L
ACGATGTGCTGCTGGGCGGCGACGGCGACGACCAGCTGTCGGGCGACGCCGGACGCGATCGCTTGTACGGCGAAGCCGGTGACGACTGGTTCTTCCAGGATGCCGCCAATGCCGGCAATC

L D G G D G R D T V D F S G P G R G L D A G A K G V F L S L G K G F A S L M D E
TGCTCGACGGCGGCGACGGCCGCGATACCGTGGATTTCAGCGGCCCGGGCCGGGGCCTCGACGCCGGCGCAAAGGGCGTATTCCTGAGCTTGGGCAAGGGGTTCGCCAGCCTGATGGACG

P E T S N V L R N I E N A V G S A R D D V L I G D A G A N V L N G L A G N D V L
AACCCGAAACCAGCAACGTGTTGCGCAATATCGAGAACGCCGTGGGCAGCGCGCGTGATGACGTGCTGATCGGCGACGCAGGCGCCAACGTCCTCAATGGCCTGGCGGGCAACGACGTGC

S G G A G D D V L L G D E G S D L L S G D A G N D D L F G G Q G D D T Y L F G V
TGTCCGGCGGCGCTGGCGACGATGTGCTGCTGGGCGACGAGGGCTCGGACCTGCTCAGCGGCGATGCGGGCAACGACGATCTGTTCGGCGGGCAGGGCGATGATACTTATCTGTTCGGGG

G Y G H D T I Y E S G G G H D T I R I N A G A D Q L W F A R Q G N D L E I R I L
TCGGGTACGGGCACGACACGATCTACGAATCGGGCGGCGGCCATGACACCATCCGCATCAACGCGGGGGCGGACCAGCTGTGGTTCGCGCGCCAGGGCAACGACCTGGAGATCCGCATTC

EP 0 366 549 A1

FIGURE 3 I

```
        G T D D A L T V H D W Y R D A D H R V E I I H A A N Q A V D Q A G I E K L V E A
        TCGGCACCGACGATGCACTTACCGTGCACGACTGGTATCGCGACGCCGATCACCGGGTGGAAATCATCCATGCCGCCAACCAGGCGGTAGACCAGGCAGGCATCGAAAAGCTGGTCGAGG
  .        5890      5900      5910      5920      5930      5940      5950      5960      5970      5980      5990      6000


        M A Q Y P D P G A A A A A P P A A R V P D T L M Q S L A V N W R *
        CAATGGCGCAGTATCCGGACCCCGGCGCGGCGGCGGCTGCCCCGCCGGCGGCGCGCGTGCCGGACACGCTGATGCAGTCCCTGGCTGTCAACTGGCGCTGAAGCGCCGTGAATCACGGCC
  .        6010      6020      6030      6040      6050      6060      6070      6080      6090      6100      6110      6120


                                       M T S P A A Q C A S V P D S G L L C L V M
        CGCCTGCCTCGCGCGGCGGCGCCGTCTCTTTGCGTTCTTCTCCGAGGTATTTCCCATCATGACGTCGCCCGCGGCGCAATGCGCCAGCGTGCCCGATTCCGGGTTGCTCTGCCTGGTCAT
  .        6130      6140      6150      6160      6170      6180      6190      6200      6210      6220      6230      6240


        L A R Y H G L A A D P E Q L R H E F A E R H S V A K R Y S L A A R R V G L K V R
        GCTGGCTCGCTATCACGGATTGGCAGCCGATCCCGAGCAGTTGCGGCATGAGTTCGCCGAGAGGCATTCTGTAGCGAAACGATACAGCCTGGCGGCGCGCCGGGTCGGCCTGAAAGTGCG
  .        6250      6260      6270      6280      6290      6300      6310      6320      6330      6340      6350      6360


        R H R P A P A R L P R A P L P A I A L D P V G R L I S
        GCGGCACCGACCCGCGCCGGCGCGCGGCTGCCACGCGCGCCGCTGCCGGCCATCGCGCTGGACCCGGTAGGGCGGCTAATTTCTT
  .        6370      6380      6390      6400      6410      6420      6430      6440        7        17        27        37
```

FIGURE 3 J

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 235 474  (INSTITUT PASTEUR) <br> * Revendications * <br> --- | 17 | C 12 N   15/31 <br> C 12 N    9/88 <br> A 61 K   39/10 |
| A | EP-A-0 275 689  (THE BOARD OF THE TRUSTEES OF THE LELAND STANFORD JUNIOR UNIVERSITY) <br> * Page 3, lignes 10-20; revendications * <br> --- | 1-19 | |
| A | SCIENCE, vol. 242, no. 4875, 7 octobre 1988, pages 72-74; W.N. BURNETTE et al.: "Pertussis toxin S1 mutant with reduced enzyme activity and a conserved protective epitope" <br> * En entier * <br> --- | 1-19 | |
| A | INFECTION AND IMMUNITY, vol. 42, no. 1, octobre 1983, pages 33-41, American Society for Microbiology; A.A. WEISS et al.: "Tn5-induced mutations affecting virulence factors of Bordetella pertussis" <br> * En entier * <br> --- | 1-19 | |
| A | THE JOURNAL OF BIOLOGICAL CHEMISTRY, vol. 261, no. 34, 5 décembre 1986, pages 16264-16269, The American Society of Biological Chemists, Inc., US; D. LADANT et al.: "Bordetella pertussis adenylate cyclase. Purification, characterization, and radioimmunoassay" <br> * En entier, en particulier page 1627, colonne 2, ligne 15 - page 1628, colonne 1, ligne 15 * <br> ---                               -/- | 1-19 | |

**DOMAINES TECHNIQUES RECHERCHES (Int. Cl.5)**

C 12 N
A 61 K

Le présent rapport a été établi pour toutes les revendications

| Lieu de la recherche | Date d'achèvement de la recherche | Examinateur |
|---|---|---|
| LA HAYE | 05-01-1990 | HUBER-MACK A. |

Office européen
des brevets

RAPPORT DE RECHERCHE EUROPEENNE

Numero de la demande

EP 89 40 2948

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | JOURNAL OF BACTERIOLOGY, vol. 170, no. 5, mai 1988, pages 2263-2266, American Society for Microbiology; M.T. TIPPETTS et al.: "Molecular cloning and expression of the Bacillus anthracis edema factor toxin gene: a calmodulin-dependent adenylate cyclase" * Page 2266, colonne 1, lignes 13-21 * | 1,10,12 | |
| P,X | THE EMBO JOURNAL, vol. 8, no. 3, p. 967-972, mar 1988; P. GLASER et al.: "Identification of residues essential for catalysis and binding of calmodulin in Bordetella pertussis adenylate cyclase by site-directed mutagenesis" * En entier * | 1-4,7-16,19 | |
| P,X | BIOCHEMISTRY, vol. 28, no., p. 2772-2776; D.C. AU et al.: "Site-direced mutagenesis of lysine 58 in a Putative ATP-binding domain of the calmodulin-sensitive adenylate cyclase from Bordetella pertussis abolishes catalytic activity" * En entier * | 1-3,7,8 ,10-15, 19 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 05-01-1990 | HUBER-MACK A. |